# EUROPEAN PATENT APPLICATION

(11) **EP 2 263 709 A1**
(43) Date of publication of application: **22.12.2010**
(21) Application number: 10182123.9
(22) Date of filing: 10.03.2003
(51) Int. Cl.: A61L 31/16, A61L 27/54, A61L 27/34, A61L 29/08, A61L 29/16, A61L 31/10

(54) **Medical device having hydration inhibitor**

(30) Priority: 06.09.2002 US 235572; 10.09.2002 WO PCT/US02/28798; 10.09.2002 WO PCT/US02/28776
(62) Divisional of application: 03714060.5
(71) Applicant: Abbott Laboratories, Abbott Park, IL 60064-6008 (US)
(72) Inventor: Cromack, Keith, R., Gurnee,, IL 60031 (US); Toner, John, L., Libertyville, IL 60048 (US)
(74) Representative: Modiano, Micaela Nadia

(57) **Abstract**

Medical devices comprising an interventional component for delivering multiple beneficial agents in which a hydration inhibitor controls release of at least one beneficial agent from the device. The hydration inhibitor is relatively less hydrophilic than the beneficial agent and preferably is a drug. Suitable beneficial agents include (I) dexamethasone, estradiol, anti-proliferative agents, anti-platelet agents, anti-inflammatory agents, anti-thrombotic agents, cytotoxic drugs, agents that inhibit cytokine or chemokine binding, cell de-differentiation inhibitors, anti-lipacdemic agents, matrix metalloproteinase inhibitors, cytostatic drugs, or combinations of these drugs, radiopaque markers, beta-carotene, tocopherols, tocotrienols, and antioxidants

## Description

### Cross Reference to Related Applications

This application is a continuation-in-part of International Patent Application No. PCT/US02/28776, filed September 10, 2002, which is continuation-in-part of United States Patent Application No. 09/950,307, filed September 10, 2001, which is a continuation-in-part of United States Application No. 09/433,001, filed November 2, 1999, now United States Patent No. 6,329,386, which is a divisional of United States Application No. 09/159,945, filed September 24, 1998, now United States Patent No. 6,015,815, incorporated herein by reference.

### Technical Field

The present invention relates to novel medical devices for delivering a beneficial agent, in which a hydration inhibitor controls release of the beneficial agent from the device. The hydration inhibitor is relatively less hydrophilic than the beneficial agent. More particularly, the present invention relates to medical devices for delivering multiple beneficial agents, e.g., drugs, one of which serves as a hydration inhibitor of the remaining beneficial agents. The present invention also relates to methods of treatment using interventional devices providing controlled delivery of a beneficial agent in a controlled manner through a hydration inhibitor that is relatively less hydrophilic than the beneficial agent.

### Background of The Invention

The compound cyclosporine (cyclosporin A) has found wide use since its introduction in the fields of organ transplantation and immunomodulation, and has brought about a significant increase in the success rate for transplantation procedures. Recently, several classes of macrocyclic compounds having potent immunomodulatory activity have been discovered. Okuhara *et al*., in European Patent Application No. 184,162, published June 11, 1986, disclose a number of macrocyclic compounds isolated from the genus *Streptomyces,* including the immunosuppressant FK-506, a 23-membered macrocyclic lactone, which was isolated from a strain of *S*. *tsukubaensis*.

Other related natural products, such as FR-900520 and FR-900523, which differ from FK-506 in their alkyl substituent at C-21, have been isolated from *S*. *hygroscopicus yakushimnaensis*. Another analog, FR-900525, produced by *S*. *tsukubaensis*, differs from FK-506 in the replacement of a pipecolic acid moiety with a proline group. Unsatisfactory side-effects associated with cyclosporine and FK-506 such as nephrotoxicity, have led to a continued search for immunosuppressant compounds having improved efficacy and safety, including an immunosuppressive agent which is effective topically, but ineffective systemically (U.S. Patent No. 5,457,111).

Rapamycin is a macrocyclic triene antibiotic produced by *Streptomyces hygroscopicus,* which was found to have antifungal activity, particularly against *Candida albicans,* both in vitro and in vivo (C. Vezina et al., J. Antibiot. 1975, 28, 721; S. N. Sehgal et al., J. Antibiot. 1975, 28, 727; H. A. Baker et al., J. Antibiot. 1978, 31, 539; U.S. Patent No. 3,929,992; and U.S. Patent No. 3,993,749).

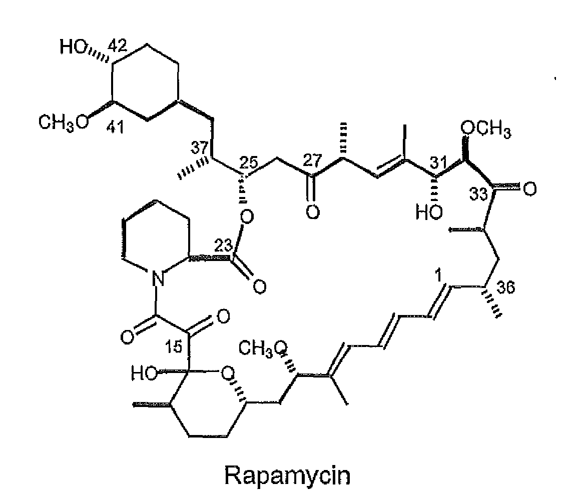

Rapamycin alone (U.S. Patent No. 4,885,171) or in combination with picibanil (U.S. Patent No. 4,401,653) has been shown to have antitumor activity. In 1977, rapamycin was also shown to be effective as an immunosuppressant in the experimental allergic encephalomyelitis model, a model for multiple sclerosis; in the adjuvant arthritis model, a model for rheumatoid arthritis; and was shown to effectively inhibit the formation of IgE-like antibodies (R. Martel et al., Can. J. Physiol. Pharmacol., 1977, 55, 48).

The immunosuppressive effects of rapamycin have also been disclosed in FASEB, 1989, 3, 3411 as has its ability to prolong survival time of organ grafts in histoincompatible rodents (R. Morris, Med. Sci. Res., 1989, 17, 877). The ability of rapamycin to inhibit T-cell activation was disclosed by M. Strauch (FASEB, 1989, 3, 3411). These and other biological effects of rapamycin are reviewed in Transplantation Reviews, 1992, 6, 39-87.

Rapamycin has been shown to reduce neointimal proliferation in animal models, and to reduce the rate of restenosis in humans. Evidence has been published showing that rapamycin also exhibits an anti-inflammatory effect, a characteristic which supported its selection as an agent for the treatment of rheumatoid arthritis. Because both cell proliferation and inflammation are thought to be causative factors in the formation of restenotic lesions after balloon angioplasty and stent placement, rapamycin and analogs thereof have been proposed for the prevention of restenosis.

Mono-ester and di-ester derivatives of rapamycin (esterification at positions 31 and 42) have been shown to be useful as antifungal agents (U.S. Patent No. 4,316,885) and as water soluble prodrugs of rapamycin (U.S. Patent No. 4,650,803).

Fermentation and purification of rapamycin and 30-demethoxy rapamycin have been described in the literature (C. Vezina et al. J. Antibiot. (Tokyo), 1975, 28 (10), 721; S. N. Sehgal et al., J. Antibiot. (Tokyo), 1975, 28(10), 727; 1983, 36(4), 351; N. L. Pavia et al., J. Natural Products, 1991, 54(1), 167-177).

Numerous chemical modifications of rapamycin have been attempted. These include the preparation of mono- and di-ester derivatives of rapamycin (WO 92/05179), 27-oximes of rapamycin (EP0 467606); 42-oxo analog of rapamycin (U.S. Patent No. 5,023,262); bicyclic rapamycins (U.S. Patent No. 5,120,725); rapamycin dimers (U.S. Patent No. 5,120,727); silyl ethers of rapamycin (U.S. Patent No. 5,120,842); and arylsulfonates and sulfamates (U.S. Patent No. 5,177, 203). Rapamycin was recently synthesized in its naturally occurring enantiomeric form (K. C. Nicolaou et al., J. Am. Chem. Soc., 1993, 115, 4419-4420; S. L. Schreiber, J. Am, Chem. Soc., 1993, 115, 7906-7907; S. J. Danishefsky, J. Am. Chem. Soc., 1993, 115, 9345-9346.

It has been known that rapamycin, like FK-506, binds to FKBP-12 (Siekierita, J. J.; Hung, S. H. Y.; Poe, M.; Lin, C. S.; Sigal, N. H. Nature, 1989, 341, 755-757; Harding, M. W.; Galat, A.; Uehling, D. E.; Schreiber, S. L. Nature 1989, 341, 758-760; Dumont, F. J.; Melino, M. R.; Staruch, M. J.; Koprak, S. L.; Fischer, P. A.; Sigal, N. H. J. Immunol. 1990, 144, 1418-1424; Bierer, B. E.; Schreiber, S. L.; Burakoff, S. J. Eur. J, Immunol. 1991, 21, 439-445; Fretz, H.; Albers, M. W.; Galat, A.; Standaert, R. F.; Lane, W. S.; Burakoff, S. J.; Bierer, B. E.; Schreiber, S. L. J. Am. Chem. Soc. 1991, 113, 1409-1411). Recently it has been discovered that the rapamycin/FKBP-12 complex binds to yet another protein, which is distinct from calcineurin, the protein that the FK-506/FKBP-12 complex inhibits (Brown, E. J.; Albers, M. W.; Shin, T. B.; Ichikawa, K.; Keith, C. T.; Lane, W. S.; Schreiber, S. L. Nature 1994, 369, 756-758; Sabatini, D. M.; Erdjument-Bromage, H.; Lui, M.; Tempest, P.; Snyder, S. H. Cell, 1994, 78, 35-43).

Percutaneous transluminal coronary angioplasty (PTCA) was developed by Andreas Gruntzig in the 1970's. The first canine coronary dilation was performed on September 24, 1975; studies showing the use of PTCA were presented at the annual meetings of the American Heart Association the following year. Shortly thereafter, the first human patient was studied in Zurich, Switzerland, followed by the first American human patients in San Francisco and New York. While this procedure changed the practice of interventional cardiology with respect to treatment of patients with obstructive coronary artery disease, the procedure did not provide long-term solutions. Patients received only temporary abatement of the chest pain associated with vascular occlusion; repeat procedures were often necessary. It was determined that the existence of restenotic lesions severely limited the usefulness of the new procedure. In the late 1980's, stents were introduced to maintain vessel patency after angioplasty. Stenting is involved in 90% of angioplasty performed today. Before the introduction of stents, the rate of restenosis ranged from 30% to 50% of the patients who were treated with balloon angioplasty. The recurrence rate after dilatation of in-stent restenosis may be as high as 70% in selected patient subsets, while the angiographic restenosis rate in de novo stent placement is about 20%. Placement of the stent reduced the restenosis rate to 15% to 20%. This percentage likely represents the best results obtainable with purely mechanical stenting. The restenosis lesion is caused primarily by neointimal hyperplasia, which is distinctly different from atherosclerotic disease both in time-course and in histopathologic appearance. Restenosis is a healing process of damaged coronary arterial walls, with neointimal tissue impinging significantly on the vessel lumen. Vascular brachytherapy appears to be efficacious against in-stent restenosis lesions. Radiation, however, has limitations of practicality and expense, and lingering questions about safety and durability.

Accordingly, it is desired to reduce the rate of restenosis by at least 50% of its current level. It is for this reason that a major effort is underway by the interventional device community to fabricate and evaluate drug-eluting stents. Such devices could have many advantages if they were successful, principally since such systems would need no auxiliary therapies, either in the form of peri-procedural techniques or chronic oral pharmacotherapy.

Drug elution rates from a drug-loaded coating containing a hydrophilic (or lipophobic) drug are usually very fast initially when the coated device contacts body fluid or blood. Thus, an ongoing problem for drug delivery stents is achieving therapeutic drug concentrations at a target site within the body with minimal losses and systemic side effects. One technique to reduce the so-called burst effect is to add a membrane containing porosigen over the coating layer containing the biologically active material, as described for example in U.S. Patent Nos. 5605696 and 5447724. Polymers are also used on stents as drug release coatings, as taught for example in US 6419692. U.S. Patent 6284305 describes elastomer coated implants in which the elastomer overcoat controls release of biologically active agent from an undercoat applied to a stent. U.S. Patent 5624411 discloses a porous polymer on a stent to control the administration of a drug. WO 0187372 describes a stent coated with a polymer loaded with a combination of drugs, such as rapamycin and dexamethasone. In these coatings, the amount of polymer is relatively high, for example about 70% of the drug-loaded coating.

Pinchuk, in U.S. Patent No. 5,092,877, discloses a stent of a polymeric material that may be employed with a coating associated with the delivery of drugs. Other patents which are directed to devices of the class utilizing bio-degradable or bio-sorbable polymers include Tang et al, U.S. Patent No. 4,916,193 and MacGregor, U.S. Patent No. 4,994,071. Sahatjian in U.S. Patent No. 5,304,121, discloses a coating applied to a stent consisting of a hydrogel polymer and a preselected drug; possible drugs include cell growth inhibitors and heparin.

A further method of making a coated intravascular stent carrying a therapeutic material in which a polymer coating is dissolved in a solvent and the therapeutic material dispersed in the solvent and the solvent thereafter evaporated is described in Berg et al, U.S. Pat. No. 5,464,650.

An article by Michael N. Helms entitled "Medical Device Design--A Systems Approach: Central Venous Catheters", 22nd International Society for the Advancement of Material and Process Engineering Technical Conference (1990) relates to polymer/drug/membrane systems for releasing heparin. Those polymer/ drug/membrane systems require two distinct layers to function. Ding et al., U.S. Patent No. 6,358,556 describes a process for coating a stent prosthesis using a biostable hydrophobic elastomer in which biologically active species are incorporated within a cured coating. In these coatings, the amount of polymer is relatively high, for example about 70% of the drug-loaded coating.

Thus, there remains a need for improved controlled delivery of a hydrophilic beneficial agent from a medical device, wherein the medical device reduces the burst effect and allows prolonged delivery of the beneficial agent without the side effects associated with some hydrophobic coatings. Also, there exist a need for a medical device with improved control of systemic release of two or more beneficial agents systematically. Further, a need exists for a medical device that is capable of releasing a beneficial agent immediately or soon after delivery followed by the controlled delivery of the same or other beneficial agents. The advantages of the present invention satisfy the aforementioned needs. Other advantages of the present invention will become apparent to those stilled in the art upon familiarization with the specification and appended claims.

### Brief Description of the Drawings

Figure 1 shows blood concentrations ± SEM (n=3) of tetrazole-containing rapamycin analogs dosed in monkey.
Figure 2 is a side view in elevation showing a stent suitable for use in this invention.
Figure 3A is a cross-sectional view of a vessel segment in which was placed a stent coated with a polymer only.
Figure 3B is a cross-sectional view of a vessel segment in which was placed a stent coated with a polymer plus drug.
Figure 4 is a cross-sectional view of a stent strut having a layer of a beneficial agent and hydration inhibitor in mixture.
Figure 5 is a cross-sectional view of a stent strut having a first layer of a beneficial agent and a second layer of a second beneficial agent acting as a hydration inhibitor.
Figure 6 is a cross-sectional view of a stent strut having a base layer of polymer material that is loaded with a mixture of a beneficial agent and a hydration inhibitor.
Figure 7 is a cross-sectional view of a stent strut having a base layer of a polymer material which is loaded with a beneficial agent and a second layer of a second beneficial agent acting as a hydration inhibitor.
Figure 8 is a cross sectional view of a stent strut having layers of a first beneficial agent alternating with layers of a second beneficial agent/hydration inhibitor.
Figures 9A and 9B illustrate top and side views of a drug-loaded coupon according to the present invention.
Figures 10, 11, 12, 13 and 14 illustrate the effect of a hydration inhibitor according to the invention on the elution of a relatively more hydrophilic beneficial agent.

### Summary of the Invention

In one aspect, the present invention generally relates to interventional devices in which delivery of a beneficial agent to be delivered from a prosthesis is controlled by an effective amount of a hydration inhibitor associated with the beneficial agent. The hydration inhibitor is relatively less hydrophilic than the beneficial agent. The present invention also relates to delivery of drug combinations, i.e., at least first and second beneficial agents. One of the beneficial agents can serve as the hydration inhibitor for another beneficial agent on the interventional device.

In one embodiment, the present invention relates to a medical device comprising an interventional component to be deployed in a patient, a beneficial agent to be delivered from the interventional component, in which the beneficial agent is loaded on at least a portion of the interventional component and has a first LogP value; and an effective amount of a hydration inhibitor associated with the beneficial agent to control delivery of the beneficial agent from the interventional component, in which the hydration inhibitor has a second LogP value, the second LogP value is greater than the first LogP value.

According to some embodiments, the beneficial agent is selected from a group consisting of antithrombotics, anticoagulants, antiplatelet agents, anti-lipid agents, thrombolytics, antiproliferatives, anti-inflammatories, agents that inhibit hyperplasia, smooth muscle cell inhibitors, antibiotics, growth factor inhibitors, cell adhesion inhibitors, cell adhesion promoters, antimitotics, antifibrins, antioxidants, antineoplastics, agents that promote endothelial cell recovery, antiallergic substances, viral vectors, nucleic acids, monoclonal antibodies, antisense compounds, oligonucleotides, cell permeation enhancers, pro-drugs and combinations thereof.

In other embodiments, the beneficial agent is selected from the group of indomethacin, phenyl salicylate, B-estradiol, vinblastine, ABT-627, testosterone, progesterone, paclitaxel, cyclosporin A, vincristine, carvedilol, vindesine, dipyridamole, methotrexate, folic acid, thrombospondin mimetics, estradiol, dexamethasone, metrizamide, iopamidol, iohexol, iopromide, iobitridol, iomeprol, iopentol, ioversol, ioxilan, iodixanol, iotrolan and pro-drugs, analogs, derivatives, or combinations thereof.

In preferred embodiments, the hydration inhibitor is a second beneficial agent.

According to some embodiments, the second beneficial agent is selected from a group consisting of antioxidants, antithrombotics, anticoagulants, antiplatelet agents, anti-lipid agents, thrombolytics, antiproliferatives, anti-inflammatories, agents that inhibit hyperplasia, smooth muscle cell inhibitors, antibiotics, growth factor inhibitors, cell adhesion inhibitors, cell adhesion promoters, antimitotics, antifibrins, antioxidants, antineoplastics, agents that promote endothelial cell recovery, antiallergic substances, viral vectors, nucleic acids, monoclonal antibodies, antisense compounds, oligonucleotides, cell permeation enhancers, radiopaque agents markers and combinations thereof

In some embodiments, the second beneficial agent is selected from a group consisting of paclitaxel, rapamycin, rapamycin derivatives, pimecrolimus, everolimus, fenofibrate, carvedilol, taxoteres, tacrolimus, butylated hydroxytoluene, butylated hydroxyanisole, vitamin E, danazol, probucol, tocopherols, tocotrienols, ABT-578, ABT-627and analogs, derivatives, or combinations thereof.

In other embodiments, the hydration inhibitor is an additive. According to these embodiments, the additive is selected from a group consisting of nitrophenyl octyl ether, bisethylhexyl sebacate, diisododecylphthalate, N-methylpyrrolidone, linolenic acid, linoleic acid stearic acid, oleic acid, and combinations thereof.

In the present invention, the hydration inhibitor is associated with the beneficial agent as a mixture of the hydration inhibitor and the beneficial agent.

The present invention also provides a method of manufacturing a medical device, in which a beneficial agent having a first LogP value is loaded on an interventional component for delivery therefrom, and an effective amount of a hydration inhibitor having a second LogP value s associated with the beneficial agent to control delivery of the beneficial agent from the interventional component, wherein the second LogP value being greater than the first LogP value.

According to the inventive methods herein, the beneficial agent is selected from a group consisting of antithrombotics, anticoagulants, antiplatelet agents, anti-lipid agents, thrombolytics, antiproliferatives, anti-inflammatories, agents that inhibit hyperplasia, smooth muscle cell inhibitors, antibiotics, growth factor inhibitors, cell adhesion inhibitors, cell adhesion promoters, antimitotics, antifibrins, antioxidants, antineoplastics, agents that promote endothelial cell recovery, antiallergic substances, viral vectors, nucleic acids, monoclonal antibodies, antisense compounds, oligonucleotides, cell permeation enhancers, and combinations thereof.

According to other embodiments, the beneficial agent loaded by the loading step is a nucleic acid, wherein the nucleic acid encodes a pharmaceutically useful peptide or an anti-sense oligo-nucleotide used to control a gene of interest in a cell of the patient.

In yet another embodiment of the present invention, the beneficial agent is selected from a group consisting indomethacin, phenyl salicylate, B-estradiol, vinblastine, ABT-627, testosterone, progesterone, paclitaxel, cyclosporin A, vincristine, carvedilol, vindesine, dipyridamole, methotrexate, folic acid, thrombospondin mimetics, estradiol, dexamethasone, metrizamide, iopamidol, iohexol, iopromide, iobitridol, iomeprol, iopentol, ioversol, ioxilan, iodixanol, iotrolan and pro-drugs, analogs, derivatives, or combinations thereof.

In a further embodiment, the second beneficial agent is selected from a group consisting of paclitaxel, rapamycin, rapamycin derivatives, pimecrolimus, everolimus, fenofibrate, carvedilol, taxoteres, tacrolimus, butylated hydroxytoluene, butylated hydroxyanisole, vitamin E, danazol, probucol, tocopherols, tocotrienols, ABT-578, ABT-627and analogs, derivatives, or combinations thereof

In one aspect of the present invention are disclosed compounds represented by the structural formula: 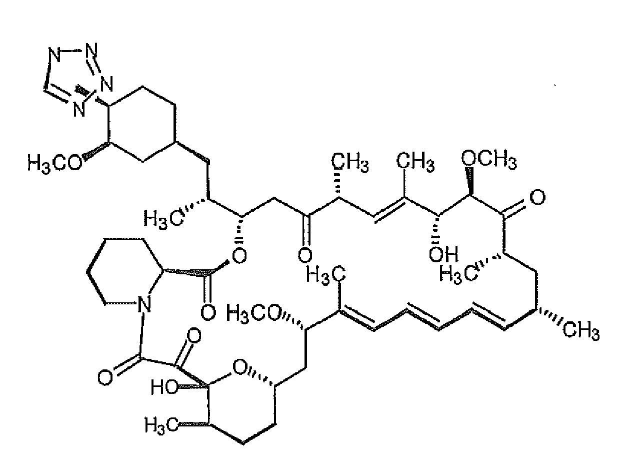 or a pharmaceutically acceptable salt or prodrug thereof.

Another object of the present invention is to provide synthetic processes for the preparation of such compounds from starting materials obtained by fermentation, as well as chemical intermediates useful in such synthetic processes.

A further object of the invention is to provide pharmaceutical compositions containing, as an active ingredient, at least one of the above compounds.

Yet another object of the invention is to provide a method of treating a variety of disease states, including restenosis, post-transplant tissue rejection, immune and autoimmune dysfunction, fungal growth, and cancer.

In another aspect this invention provides a medical device comprising a supporting structure having a coating on the surface thereof, the coating containing a therapeutic substance, such as, for example, a drug. Supporting structures for the medical devices that are suitable for use in this invention include, but are not limited to, coronary stents, peripheral stents, catheters, arterio-venous grafts, by-pass grafts, and drug delivery balloons used in the vasculature.

Drugs that are suitable for use in this invention include, but are not limited to, 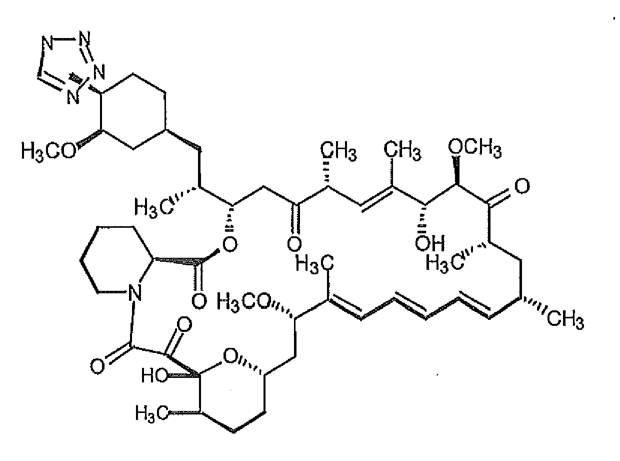 or a pharmaceutically acceptable salt or prodrug thereof, which includes 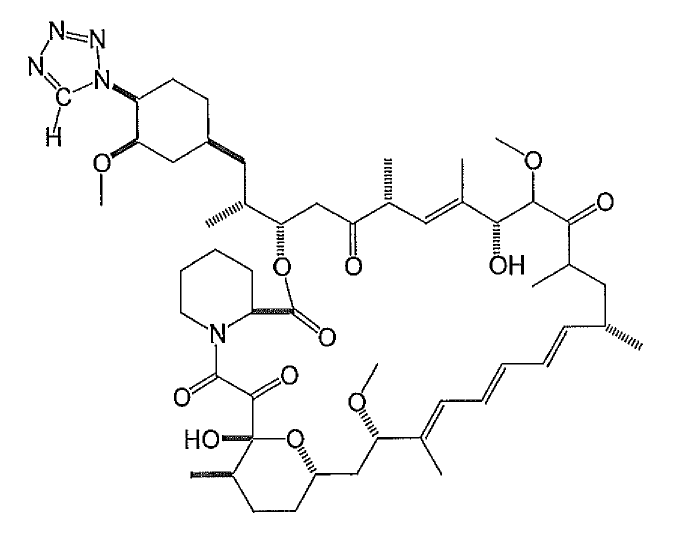 or a pharmaceutically acceptable salt or prodrug thereof, (hereinafter alternatively referred to as A-179578 or ABT-578), and 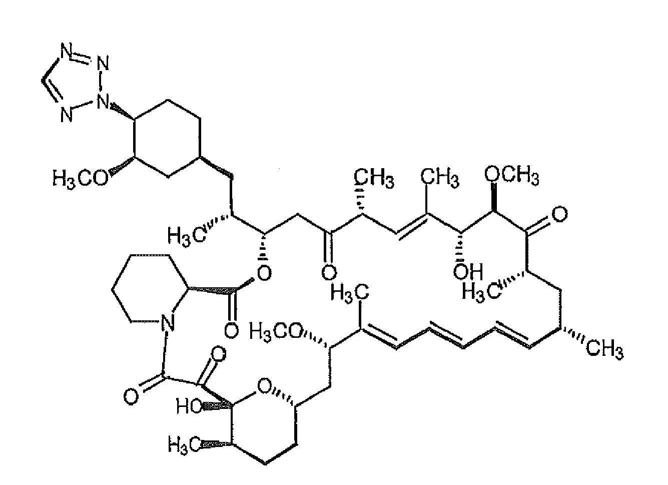 or a pharmaceutically acceptable salt or prodrug thereof; 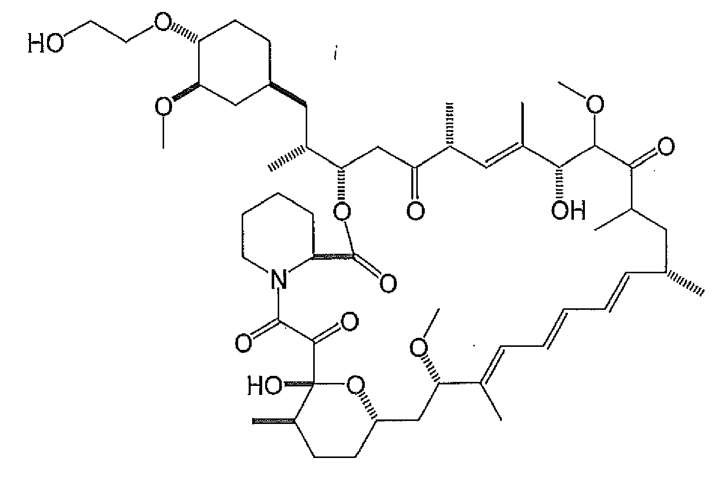 or a pharmaceutically acceptable salt or prodrug thereof, (hereinafter alternatively referred to as SDZ RAD or 40-O-(2-hydroxyethyl)-rapamycin); 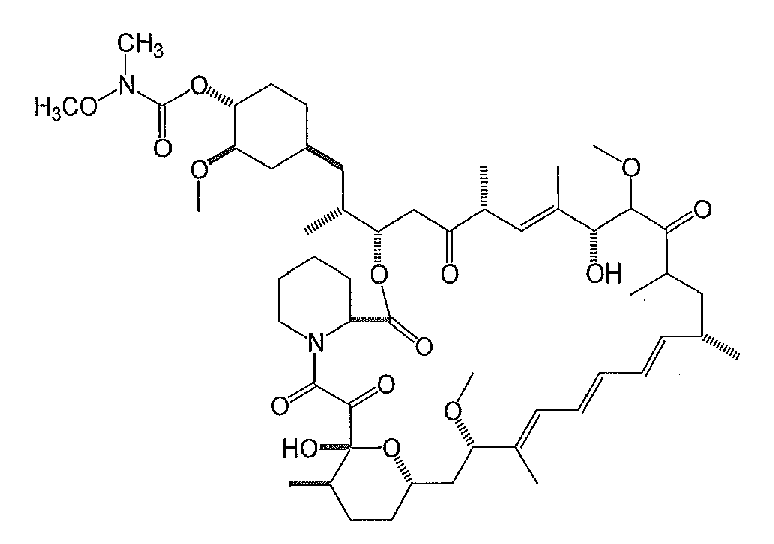 or a pharmaceutically acceptable salt or prodrug thereof, (hereinafter alternatively referred to as A-94507).

Coatings that are suitable for use in this invention include, but are not limited to, polymeric coatings that can comprise any polymeric material in which the therapeutic agent, i.e., the drug, is substantially soluble. The coating can be hydrophilic, hydrophobic, biodegradable, or non-biodegradable. This medical device reduces restenosis in vasculature. The direct coronary delivery of a drug such as A-179578 is expected to reduce the rate of restenosis to a level of about 0% to 25%.

### Detailed Description of the Invention

### Definition of Terms

The term "prodrug," as used herein, refers to compounds which are rapidly transformed in vivo to the parent compound of the above formula, for example, by hydrolysis in blood. A thorough discussion is provided by T. Higuchi and V. Stella, "Pro-drugs as Novel Delivery systems," Vol. 14 of the A. C. S. Symposium Series, and in Edward B. Roche, ed., "Bioreversible Carriers in Drug Design," American Pharmaceutical Association and Pergamon Press, 1987, both of which are incorporated herein by reference.

The term "pharmaceutically acceptable prodrugs", as used herein, refers to those prodrugs of the compounds of the present invention which are, within the scope of sound medical judgment, suitable for use in contact with the tissues of humans and lower mammals without undue toxicity, irritation, and allergic response, are commensurate with a reasonable benefit/risk ratio, and are effective for their intended use, as well as the zwitterionic forms, where possible, of the compounds of the invention. Particularly preferred pharmaceutically acceptable prodrugs of this invention are prodrug esters of the C-31 hydroxyl group of compounds of this invention.

The term "prodrug esters," as used herein, refers to any of several ester-forming groups that are hydrolyzed under physiological conditions. Examples of prodrug ester groups include acetyl, ethanoyl, pivaloyl, pivaloyloxymethyl, acetoxymethyl, phthalidyl, methoxymethyl, indanyl, and the like, as well as ester groups derived from the coupling of naturally or unnaturally-occurring amino acids to the C-31 hydroxyl group of compounds of this invention.

The term "supporting structure" means a framework that is capable of containing or supporting a pharmaceutically acceptable carrier or excipient, which carrier or excipient may contain one or more therapeutic agents or substances, e.g., one or more drugs and/or other compounds. The supporting structure is typically formed of metal or a polymeric material. Suitable supporting structures formed of polymeric materials, including biodegradable polymers, capable of containing the therapeutic agents or substances include, without limitation, those disclosed in U.S. Patent Nos. 6,413,272 and 5,527,337, which are incorporated herein by reference. Supporting structures useful in the present invention are generally part of medical devices, including implants and prostheses.

### Embodiments

In one embodiment of the invention is a compound of formula

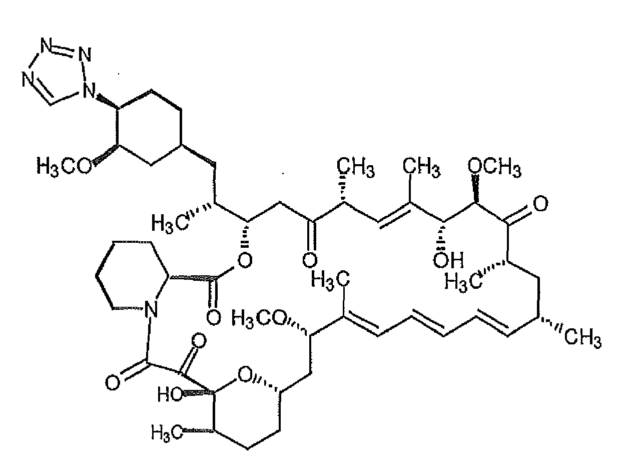

In another embodiment of the invention is a compound of formula

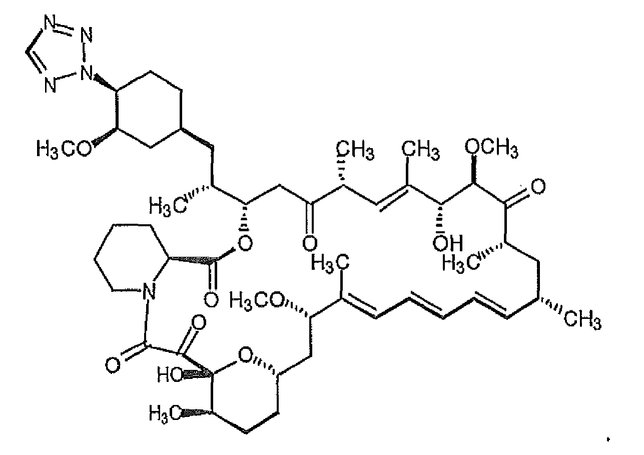

### Preparation of Compounds of this Invention

The compounds and processes of the present invention will be better understood in connection with the following synthetic schemes which illustrate the methods by which the compounds of the invention may be prepared.

The compounds of this invention may be prepared by a variety of synthetic routes. A representative procedure is shown in Scheme 1.

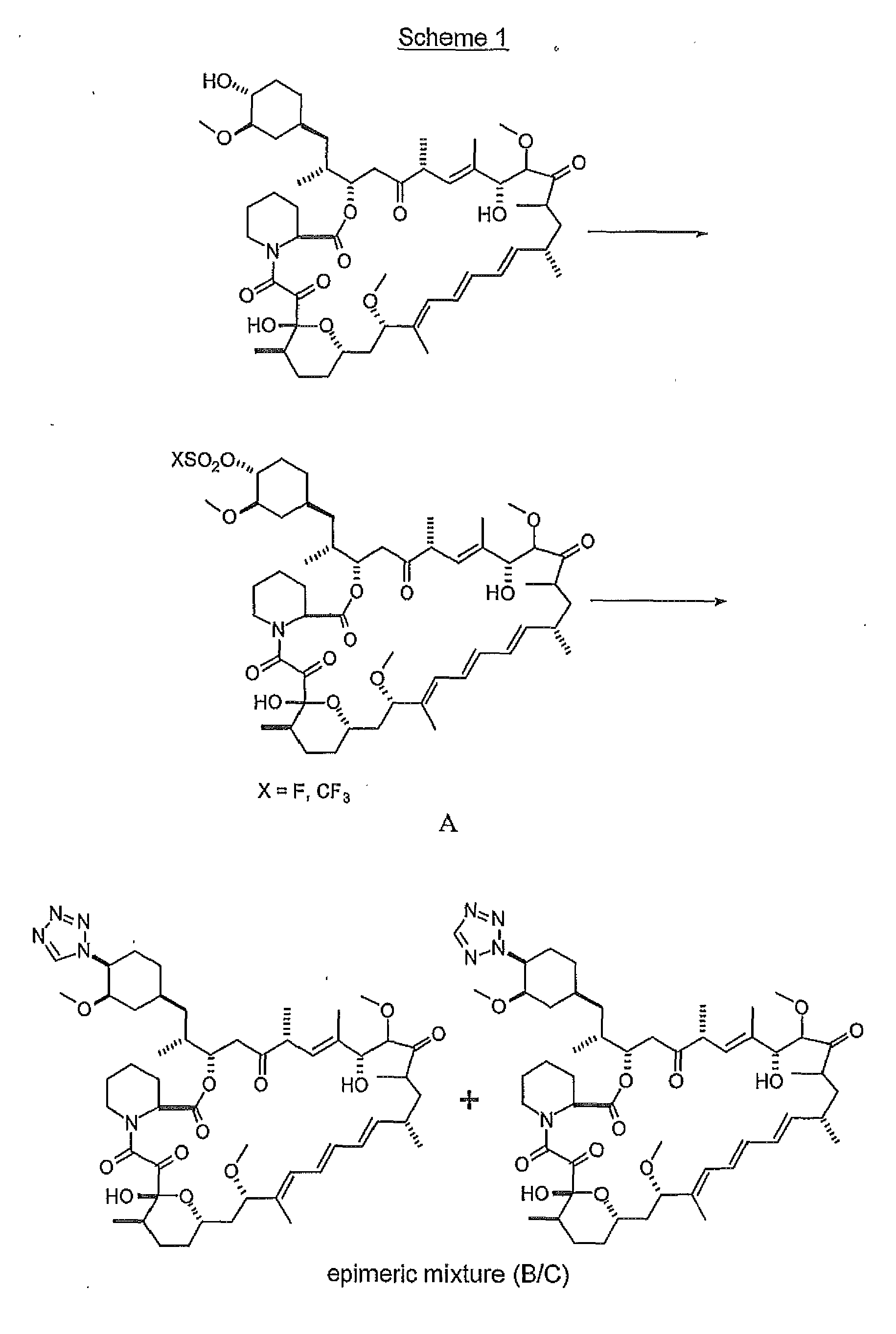

As shown in Scheme 1, conversion of the C-42 hydroxyl of rapamycin to a trifluoromethanesulfonate or fluorosulfonate leaving group provided A. Displacement of the leaving group with tetrazole in the presence of a hindered, non-nucleophilic base, such as 2,6-lutidine, or, preferably, diisopropylethyl amine provided epimers B and C, which were separated and purified by flash column chromatography.

### Synthetic Methods

The foregoing may be better understood by reference to the following examples which illustrate the methods by which the compounds of the invention may be prepared and are not intended to limit the scope of the invention as defined in the appended claims.

### Example 1

### 42-Epi-(tetrazolyl)-rapamycin (less polar isomer)

### Example 1A

A solution of rapamycin (100 mg, 0.11 mmol) in dichloromethane (0.6 mL) at - 78 °C under a nitrogen atmosphere was treated sequentially with 2,6-lutidine (53 uL, 0.46 mmol, 4.3 eq.) and trifluoromethanesulfonic anhydride (37 uL, 0.22 mmol), and stirred thereafter for 15 minutes, warmed to room temperature and eluted through a pad of silica gel (6 mL) with diethyl ether. Fractions containing the triflate were pooled and concentrated to provide the designated compound as an amber foam.

### Example 1 B

### 42-Epi-(tetrazolyl)-rapamycin (less polar isomer)

A solution of Example 1A in isopropyl acetate (0.3 mL) was treated sequentially with diisopropylethylamine (87 mL, 0.5 mmol) and 1H-tetrazole (35 mg, 0.5 mmol), and thereafter stirred for 18 hours. This mixture was partitioned between water (10 mL) and ether (10 mL). The organics were washed with brine (10 mL) and dried (Na₂SO₄). Concentration of the organics provided a sticky yellow solid which was purified by chromatography on silica gel (3.5 g, 70-230 mesh) eluting with hexane (10 mL), hexane:ether (4:1(10 mL), 3:1(10 mL), 2:1 (10 mL), 1:1(10 mL)), ether (30 mL), hexane:acetone (1:1(30mL)). One of the isomers was collected in the ether fractions.
MS (ESI) m/e 966 (M)⁻;

### Example 2

### 42-Epi-(tetrazolyl)-rapamycin (more polar isomer)

### Example 2A

### 42-Epi-(tetrazolyl)-rapamycin (more polar isomer)

Collection of the slower moving band from the chromatography column using the hexane:acetone (1:1) mobile phase in Example 1B provided the designated compound.
MS (ESI) m/e 966 (M)-.

### In vitro Assay of Biological Activity

The immunosuppressant activity of the compounds of the present invention was compared to rapamycin and two rapamycin analogs: 40-epi-N-[2'-pyridone]-rapamycin and 40-epi-N-[4'-pyridone]-rapamycin, both disclosed in U. S. Patent No. 5,527,907. The activity was determined using the human mixed lymphocyte reaction (MLR) assay described by Kino, T. et al. in Transplantation Proceedings, XIX(5):36-39, Suppl. 6 (1987). The results of the assay demonstrate that the compounds of the invention are effective immunomodulators at nanomolar concentrations, as shown in Table 1.

**Table 1**

| Example | Human MLR IC₅₀ ± S.E.M.(nM) |
|---|---|
| Rapamycin | 0.91 ± 0.36 |
| 2-pyridone | 12.39 ± 5.3 |
| 4-pyridone | 0.43 ± 0.20 |
| Example 1 | 1.70 ± 0.48 |
| Example 2 | 0.66 ± 0.19 |

The pharmacokinetic behaviors of Example 1 and Example 2 were characterized following a single 2.5 mg/kg intravenous dose in cynomolgus monkey (n=3 per group). Each compound was prepared as 2.5 mg/mL solution in a 20% ethanol:30% propylene glycol:2% cremophor EL:48% dextrose 5% in water vehicle. The 1 mL/kg intravenous dose was administered as a slow bolus (∼1-2 minutes) in a saphenous vein of the monkeys. Blood samples were obtained from a femoral artery or vein of each animal prior to dosing and 0.1 (IV only), 0.25, 0.5, 1, 1.5, 2, 4, 6, 9, 12, 24, and 30 hours after dosing. The EDTA preserved samples were thoroughly mixed and extracted for subsequent analysis.

An aliquot of blood (1.0 mL) was hemolyzed with 20% methanol in water (0.5 ml) containing an internal standard. The hemolyzed samples were extracted with a mixture of ethyl acetate and hexane (1:1 (v/v), 6.0 mL). The organic layer was evaporated to dryness with a stream of nitrogen at room temperature. Samples were reconstituted in methanol: water (1:1,150 µL). The title compounds (50 µL injection) were separated from contaminants using reverse phase HPLC with UV detection. Samples were kept cool (4 °C) through the run. All samples from each study were analyzed as a single batch on the HPLC.

Area under the curve (AUC) measurements of Example 1, Example 2 and the internal standard were determined using the Sciex MacQuan™ software. Calibration curves were derived from peak area ratio (parent drug/internal standard) of the spiked blood standards using least squares linear regression of the ratio versus the theoretical concentration. The methods were linear for both compounds over the range of the standard curve (correlation > 0.99) with an estimated quantitation limit of 0.1 ng/mL. The maximum blood concentration (C_{MAX}) and the time to reach the maximum blood concentration (T_{MAX}) were read directly from the observed blood concentration-time data. The blood concentration data were submitted to multi-exponential curve fitting using CSTRIP to obtain estimates of pharmacokinetic parameters. The estimated parameters were further defined using NONLIN84. The area under the blood concentration-time curve from 0 to t hours (last measurable blood concentration time point) after dosing (AUC₀₋ₜ) was calculated using the linear trapeziodal rule for the blood-time profiles. The residual area extrapolated to infinity, determined as the final measured blood concentration (Cₜ) divided by the terminal elimination rate constant (β), and added to AUC₀₋ₜ to produce the total area under the curve (AUC₀₋ₜ).

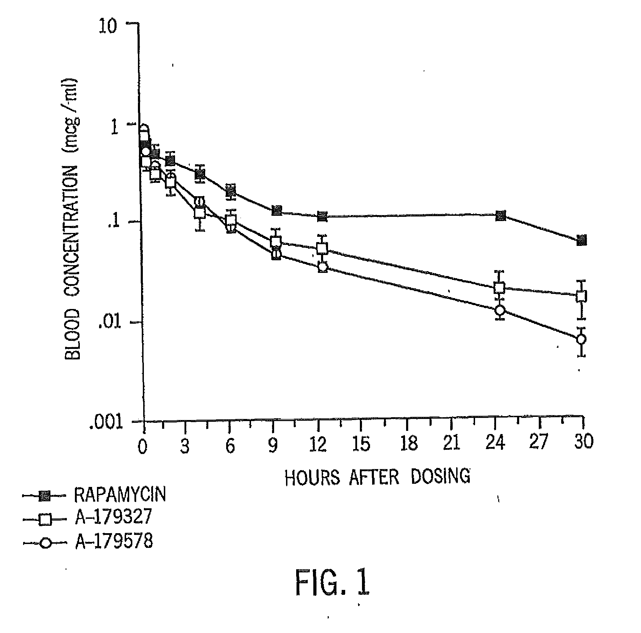

As shown in Figure 1 and Table 2, both Example 1 and Example 2 had a surprisingly substantially shorter terminal elimination half-life (t_{1/2}) when compared to rapamycin. Thus, only the compounds of the invention provide both sufficient efficacy (Table 1) and a shorter terminal half-life (Table 2).

**Table 2**

| Compound | AUC ng·hr/mL | t_{1/2} (hours) |
|---|---|---|
| Rapamycin | 6.87 | 16.7 |
| 2-pyridone | 2.55 | 2.8 |
| 4-pyridone | 5.59 | 13.3 |
| Example 1 | 2.35 | 5.0 |
| Example 2 | 2.38 | 6.9 |

### Methods of Treatment

The compounds of the invention, including but not limited to those specified in the examples, possess immunomodulatory activity in mammals (especially humans). As immunosuppressants, the compounds of the present invention are useful for the treatment and prevention of immune-mediated diseases such as the resistance by transplantation of organs or tissue such as heart, kidney, liver, medulla ossium, skin, cornea, lung, pancreas, intestinum tenue, limb, muscle, nerves, duodenum, small-bowel, pancreatic-islet-cell, and the like; graft-versus-host diseases brought about by medulla ossium transplantation; autoimmune diseases such as rheumatoid arthritis, systemic lupus erythematosus, Hashimoto's thyroiditis, multiple sclerosis, myasthenia gravis, type I diabetes, uveitis, allergic encephalomyelitis, glomerulonephritis, and the like. Further uses include the treatment and prophylaxis of inflammatory and hyperproliferative skin diseases and cutaneous manifestations of immunologically-mediated illnesses, such as psoriasis, atopic dermatitis, contact dermatitis and further eczematous dermatitises, seborrhoeis dermatitis, lichen planus, pemphigus, bullous pemphigoid, epidermolysis bullosa, urticaria, angioedemas, vasculitides, erythemas, cutaneous eosinophillas, lupus erythematosus, acne and alopecia areata; various eye diseases (autoimmune and otherwise) such as keratoconjunctivitis, vernal conjunctivitis, uveitis associated with Behcet's disease, keratitis, herpetic keratitis, conical cornea, dystrophia epithelialis corneae, corneal leukoma, and ocular pemphigus. In addition reversible obstructive airway disease, which includes conditions such as asthma (for example, bronchial asthma, allergic asthma, intrinsic asthma, extrinsic asthma and dust asthma), particularly chronic or inveterate asthma (for example, late asthma and airway hyper-responsiveness), bronchitis, allergic rhinitis, and the like are targeted by compounds of this invention. Inflammation of mucosa and blood vessels such as gastric ulcers, vascular damage caused by ischemic diseases and thrombosis. Moreover, hyperproliferative vascular diseases such as intimal smooth muscle cell hyperplasia, restenosis and vascular occlusion, particularly following biologically- or mechanically-mediated vascular injury, could be treated or prevented by the compounds of the invention.

The compounds or drugs described herein can be applied to stents that have been coated with a polymeric compound. Incorporation of the compound or drug into the polymeric coating of the stent can be carried out by dipping the polymer-coated stent into a solution containing the compound or drug for a sufficient period of time (such as, for example, five minutes) and then drying the coated stent, preferably by means of air drying for a sufficient period of time (such as, for example, 30 minutes). The polymer-coated stent containing the compound or drug can then be delivered to the coronary vessel by deployment from a balloon catheter. In addition to stents, other devices that can be used to introduce the drugs of this invention to the vasculature include, but are not limited to grafts, catheters, and balloons. In addition, other compounds or drugs that can be used in lieu of the drugs of this invention include, but are not limited to, A-94507 and SDZ RAD).

The compounds described herein for use in polymer-coated stents can be used in combination with other pharmacological agents. The pharmacological agents that would, in combination with the compounds of this invention, be most effective in preventing restenosis can be classified into the categories of anti-proliferative agents, anti-platelet agents, anti-inflammatory agents, anti-thrombotic agents, and thrombolytic agents. These classes can be further sub-divided. For example, anti-proliferative agents can be anti-mitotic. Anti-mitotic agents inhibit or affect cell division, whereby processes normally involved in cell division do not take place. One sub-class of anti-mitotic agents includes vinca alkaloids. Representative examples of vinca alkaloids include, but are not limited to, vincristine, paclitaxel, etoposide, nocodazole, indirubin, and anthracycline derivatives, such as, for example, daunorubicin, daunomycin, and plicamycin. Other sub-classes of anti-mitotic agents include anti-mitotic alkylating agents, such as, for example, tauromustine, bofumustine, and fotemustine, and anti-mitotic metabolites, such as, for example, methotrexate, fluorouracil, 5-bromodeoxyuridine, 6-azacytidine, and cytarabine. Anti-mitotic alkylating agents affect cell division by covalently modifying DNA, RNA, or proteins, thereby inhibiting DNA replication, RNA transcription, RNA translation, protein synthesis, or combinations of the foregoing.

Anti-platelet agents are therapeutic entities that act by (1) inhibiting adhesion of platelets to a surface, typically a thrombogenic surface, (2) inhibiting aggregation of platelets, (3) inhibiting activation of platelets, or (4) combinations of the foregoing. Activation of platelets is a process whereby platelets are converted from a quiescent, resting state to one in which platelets undergo a number of morphologic changes induced by contact with a thrombogenic surface. These changes include changes in the shape of the platelets, accompanied by the formation of pseudopods, binding to membrane receptors, and secretion of small molecules and proteins, such as, for example, ADP and platelet factor 4. Anti-platelet agents that act as inhibitors of adhesion of platelets include, but are not limited to, eptifibatide, tirofiban, RGD (Arg-Gly-Asp)-based peptides that inhibit binding to gpIIbIIIa or αvβ3, antibodies that block binding to gpIIaIIIb or αvβ3, anti-P-selectin antibodies, anti-E-selectin antibodies, peptides that block P-selectin or E-selectin binding to their respective ligands, saratin, and anti-von Willebrand factor antibodies. Agents that inhibit ADP-mediated platelet aggregation include, but are not limited to, disagregin and cilostazol.

Anti-inflammatory agents can also be used. Examples of these include, but are not limited to, prednisone, dexamethasone, hydrocortisone, estradiol, and non-steroidal anti-inflammatories, such as, for example, acetaminophen, ibuprofen, naproxen, and sulindac. Other examples of these agents include those that inhibit binding of cytokines or chemokines to the cognate receptors to inhibit pro-inflammatory signals transduced by the cytokines or the chemokines. Representative examples of these agents include, but are not limited to, anti-IL1, anti-IL2, anti-IL3, anti-IL4, anti-IL8, anti-IL15, anti-GM-CSF, and anti-TNF antibodies.

Anti-thrombotic agents include chemical and biological entities that can intervene at any stage in the coagulation pathway. Examples of specific entities include, but are not limited to, small molecules that inhibit the activity of factor Xa. In addition, heparinoid-type agents that can inhibit both FXa and thrombin, either directly or indirectly, such as, for example, heparin, heparan sulfate, low molecular weight heparins, such as, for example, the compound having the trademark Clivarin®, and synthetic oligosaccharides, such as, for example, the compound having the trademark Arixtra®. Also included are direct thrombin inhibitors, such as, for example, melagatran, ximelagatran, argatroban, inogatran, and peptidomimetics of binding site of the Phe-Pro-Arg fibrinogen substrate for thrombin. Another class of anti-thrombotic agents that can be delivered are factor VII/VIIa inhibitors, such as, for example, anti-factor VII/VIIa antibodies, rNAPc2, and tissue factor pathway inhibitor (TFPI).

Thrombolytic agents, which may be defined as agents that help degrade thrombi (clots), can also be used as adjunctive agents, because the action of lysing a clot helps to disperse platelets trapped within the fibrin matrix of a thrombus. Representative examples of thrombolytic agents include, but are not limited to, urokinase or recombinant urokinase, pro-urokinase or recombinant pro-urokinase, tissue plasminogen activator or its recombinant form, and streptokinase.

Other drugs that can be used in combination with the compounds of this invention are cytotoxic drugs, such as, for example, apoptosis inducers, such as TGF, and topoisomerase inhibitors, such as, 10-hydroxycamptothecin, irinotecan, and doxorubicin. Other classes of drugs that can be used in combination with the compounds of this invention are drugs that inhibit cell de-differentiation and cytostatic drugs.

Other agents that can be used in combination with the compounds of this invention include anti-lipaedemic agents, such as, for example, fenofibrate, matrix metalloproteinase inhibitors, such as, for example, batimistat, antagonists of the endothelin-A receptor, such as, for example, darusentan, and antagonists of the αvβ3 integrin receptor.

When used in the present invention, the coating can comprise any polymeric material in which the therapeutic agent, i.e., the drug, is substantially soluble. The purpose of the coating is to serve as a controlled release vehicle for the therapeutic agent or as a reservoir for a therapeutic agent to be delivered at the site of a lesion. The coating can be polymeric and can further be hydrophilic, hydrophobic, biodegradable, or non-biodegradable. The material for the polymeric coating can be selected from the group consisting of polycarboxylic acids, cellulosic polymers, gelatin, polyvinylpyrrolidone, maleic anhydride polymers, polyamides, polyvinyl alcohols, polyethylene oxides, glycosaminoglycans, polysaccharides, polyesters, polyurethanes, silicones, polyorthoesters, polyanhydrides, polycarbonates, polypropylenes, polylactic acids, polyglycolic acids, polycaprolactones, polyhydroxybutyrate valerates, polyacrylamides, polyethers, and mixtures and copolymers of the foregoing. Coatings prepared from polymeric dispersions such as polyurethane dispersions (BAYHYDROL, etc.) and acrylic acid latex dispersions can also be used with the therapeutic agents of the present invention.

Biodegradable polymers that can be used in this invention include polymers such as poly(L-lactic acid), poly(DL-lactic acid), polycaprolactone, poly(hydroxy butyrate), polyglycolide, poly(diaxanone), poly(hydroxy valerate), polyorthoester; copolymers such as poly (lactide-co-glycolide), polyhydroxy (butyrate-co-valerate), polyglycolide-co-trimethylene carbonate; polyanhydrides; polyphosphoester; polyphosphoester-urethane; polyamino acids; polycyanoacrylates; biomolecules such as fibrin, fibrinogen, cellulose, starch, collagen and hyaluronic acid; and mixtures of the foregoing. Biostable materials that are suitable for use in this invention include polymers such as polyurethane, silicones, polyesters, polyolefins, polyamides, polycaprolactam, polyimide, polyvinyl chloride, polyvinyl methyl ether, polyvinyl alcohol, acrylic polymers and copolymers, polyacrylonitrile, polystyrene copolymers of vinyl monomers with olefins (such as styrene acrylonitrile copolymers, ethylene methyl methacrylate copolymers, ethylene vinyl acetate), polyethers, rayons, cellulosics (such as cellulose acetate, cellulose nitrate, cellulose propionate, etc.), parylene and derivatives thereof; and mixtures and copolymers of the foregoing.

Another polymer that can be used in this invention is poly(MPC_{w}:LAMₓ:NPMA_{y}:TSMA_{z}) where w, x, y, and z represent the molar ratios of monomers used in the feed for preparing the polymer and MPC represents the unit 2-methacryoyloxyethylphosphorylcholine, LMA represents the unit lauryl methacrylate, HPMA represents the unit 2-hydroxypropyl methacrylate, and TSMA represents the unit 3-trimethoxysilylpropyl methacrylate. The drug-impregnated stent can be used to maintain patency of a coronary artery previously occluded by thrombus and/or atherosclerotic plaque. The delivery of an anti-proliferative agent reduces the rate of in-stent restenosis.

Other treatable conditions include but are not limited to ischemic bowel diseases, inflammatory bowel diseases, necrotizing enterocolitis, intestinal inflammations/allergies such as Coeliac diseases, proctitis, eosinophilic gastroenteritis, mastocytosis, Crohn's disease and ulcerative colitis; nervous diseases such as multiple myositis, Guillain-Barre syndrome, Meniere's disease, polyneuritis, multiple neuritis, mononeuritis and radiculopathy; endocrine diseases such as hyperthyroidism and Basedow's disease; hematic diseases such as pure red cell aplasla, aplastic anemia, hypoplastic anemia, idiopathic thrombocytopenic purpura, autoimmune hemolytic anemia, agranulocytosis, pernicious anemia, megaloblastic anemia and anerythroplasia; bone diseases such as osteoporosis; respiratory diseases such as sarcoidosis, fibroid lung and idiopathic interstitial pneumonia; skin disease such as dermatomyositis, leukoderma vulgaris, ichthyosis vulgaris, photoallergic sensitivity and cutaneous T cell lymphoma; circulatory diseases such as arteriosclerosis, atherosclerosis, aortitis syndrome, polyarteritis nodosa and myocardosis; collagen diseases such as scleroderma, Wegener's granuloma and Sjogren's syndrome; adiposis; eosinophilic fasciitis; periodontal disease such as lesions of gingiva, periodontium, alveolar bone and substantia ossea dentis; nephrotic syndrome such as glomerulonephritis; male pattern alopecia or alopecia senilis by preventing epilation or providing hair germination and/or promoting hair generation and hair growth; muscular dystrophy; Pyoderma and Sezary's syndrome; Addison's disease; active oxygen-mediated diseases, as for example organ injury such as ischemia-reperfusion injury of organs (such as heart, liver, kidney and digestive tract) which occurs upon preservation, transplantation or ischemic disease (for example, thrombosis and cardiac infarction); intestinal diseases such as endotoxin-shock, pseudomembranous colitis and colitis caused by drug or radiation; renal diseases such as ischemic acute renal insufficiency and chronic renal insufficiency; pulmonary diseases such as toxinosis caused by lung-oxygen or drug (for example, paracort and bleomycins), lung cancer and pulmonary emphysema; ocular diseases such as cataracta, siderosis, retinitis, pigmentosa, senile macular degeneration, vitreal scarring and corneal alkali burn; dermatitis such as erythema multiforme, linear IgA ballous dermatitis and cement dermatitis; and others such as gingivitis, periodontitis, sepsis, pancreatitis, diseases caused by environmental pollution (for example, air pollution), aging, carcinogenesis, metastasis of carcinoma and hypobaropathy; diseases caused by histamine or leukotriene-C₄ release; Behcet's disease such as intestinal-, vasculo- or neuro-Behcet's disease, and also Behcet's which affects the oral cavity, skin, eye, vulva, articulation, epididymis, lung, kidney and so on. Furthermore, the compounds of the invention are useful for the treatment and prevention of hepatic disease such as immunogenic diseases (for example, chronic autoimmune liver diseases such as autoimmune hepatitis, primary biliary cirrhosis and sclerosing cholangitis), partial liver resection, acute liver necrosis (e.g. necrosis caused by toxin, viral hepatitis, shock or anoxia), B-virus hepatitis, non-A/non-B hepatitis, cirrhosis (such as alcoholic cirrhosis) and hepatic failure such as fulminant hepatic failure, late-onset hepatic failure and "acute-on-chronic" liver failure (acute liver failure on chronic liver diseases), and moreover are useful for various diseases because of their useful activity such as augmentation of chemotherapeutic effect, cytomegalovirus infection, particularly HCMV infection, anti-inflammatory activity, sclerosing and fibrotic diseases such as nephrosis, scleroderma, pulmonary fibrosis, arteriosclerosis, congestive heart failure, ventricular hypertrophy, post-surgical adhesions and scarring, stroke, myocardial infarction and injury associated with ischemia and reperfusion, and the like.

Additionally, compounds of the invention possess FK-506 antagonistic properties. The compounds of the present invention may thus be used in the treatment of immunodepression or a disorder involving immunodepression. Examples of disorders involving immunodepression include AIDS, cancer, fungal infections, senile dementia, trauma (including wound healing, surgery and shock) chronic bacterial infection, and certain central nervous system disorders. The immunodepression to be treated may be caused by an overdose of an immunosuppressive macrocyclic compound, for example derivatives of 12-(2-cyclohexyl-1-methylvinyl)-13, 19,21,27-tetramethyl-11,28-dioxa-4-azatricyclo[22.3.1.0 ^{4,9}] octacos-18-ene such as FK-506 or rapamycin. The overdosing of such medicaments by patients is quite common upon their realizing that they have forgotten to take their medication at the prescribed time and can lead to serious side effects.

The ability of the compounds of the invention to treat proliferative diseases can be demonstrated according to the methods described in Bunchman ET and CA Brookshire, Transplantation Proceed. 23 967-968 (1991); Yamagishi, et al., Biochem, Biophys. Res. Comm, 191 840-846 (1993); and Shichiri, et al., J. Clin. Invest. 87 1867-1871 (1991). Proliferative diseases include smooth muscle proliferation, systemic sclerosis, cirrhosis of the liver, adult respiratory distress syndrome, idiopathic cardiomyopathy, lupus erythematosus, diabetic retinopathy or other retinopathies, psoriasis, scleroderma, prostatic hyperplasia, cardiac hyperplasia, restenosis following arterial injury or other pathologic stenosis of blood vessels. In addition, these compounds antagonize cellular responses to several growth factors, and therefore possess antiangiogenic properties, making them useful agents to control or reverse the growth of certain tumors, as well as fibrotic diseases of the lung, liver, and kidney.

Aqueous liquid compositions of the present invention are particularly useful for the treatment and prevention of various diseases of the eye such as autoimmune diseases (including, for example, conical cornea, keratitis, dysophia epithelialis corneae, leukoma, Mooren's ulcer, sclevitis and Graves' ophthalmopathy) and rejection of corneal transplantation.

When used in the above or other treatments, a therapeutically effective amount of one of the compounds of the present invention may be employed in pure form or, where such forms exist, in pharmaceutically acceptable salt, ester or prod rug form. Alternatively, the compound may be administered as a pharmaceutical composition containing the compound of interest in combination with one or more pharmaceutically acceptable excipients. The phrase "therapeutically effective amount" of the compound of the invention means a sufficient amount of the compound to treat disorders, at a reasonable benefit/risk ratio applicable to any medical treatment. It will be understood, however, that the total daily usage of the compounds and compositions of the present invention will be decided by the attending physician within the scope of sound medical judgment. The specific therapeutically effective dose level for any particular patient will depend upon a variety of factors including the disorder being treated and the severity of the disorder; activity of the specific compound employed; the specific composition employed; the age, body weight, general health, sex and diet of the patient; the time of administration, route of administration, and rate of excretion of the specific compound employed; the duration of the treatment; drugs used in combination or coincidental with the specific compound employed; and like factors well known in the medical arts. For example, it is well within the skill of the art to start doses of the compound at levels lower than required to achieve the desired therapeutic effect and to gradually increase the dosage until the desired effect is achieved.

The total daily dose of the compounds of this invention administered to a human or lower animal may range from about 0.01 to about 10 mg/kg/day. For purposes of oral administration, more preferable doses may be in the range of from about 0.001 to about 3 mg/kg/day. For the purposes of local delivery from a stent, the daily dose that a patient will receive depends on the length of the stent. For example, a 15 mm coronary stent may contain a drug in an amount ranging from about 1 to about 120 micrograms and may deliver that drug over a time period ranging from several hours to several weeks. If desired, the effective daily dose may be divided into multiple doses for purposes of administration; consequently, single dose compositions may contain such amounts or submultiples thereof to make up the daily dose. Topical administration may involve doses ranging from 0.001 to 3% mg/kg/day, depending on the site of application.

### Pharmaceutical Compositions

The pharmaceutical compositions of the present invention comprise a compound of the invention and a pharmaceutically acceptable carrier or excipient, which may be administered orally, rectally, parenterally, intracisternally, intravaginally, intraperitoneally, topically (as by powders, ointments, drops or transdermal patch), bucally, as an oral or nasal spray, or locally, as in a stent placed within the vasculature. The phrase "pharmaceutically acceptable carrier" means a non-toxic solid, semi-solid or liquid filler, diluent, encapsulating material or formulation auxiliary of any type. The term "parenteral," as used herein, refers to modes of administration which include intravenous, intraarterial, intramuscular, intraperitoneal, intrasternal, subcutaneous and intraarticular injection, infusion, and placement, such as , for example, in vasculature.

Pharmaceutical compositions of this invention for parenteral injection comprise pharmaceutically acceptable sterile aqueous or nonaqueous solutions, dispersions, suspensions or emulsions as well as sterile powders for reconstitution into sterile injectable solutions or dispersions just prior to use. Examples of suitable aqueous and nonaqueous carriers, diluents, solvents or vehicles include water, ethanol, polyols (such as glycerol, propylene glycol, polyethylene glycol, and the like), carboxymethylcellulose and suitable mixtures thereof, vegetable oils (such as olive oil), and injectable organic esters such as ethyl oleate. Proper fluidity can be maintained, for example, by the use of coating materials such as lecithin, by the maintenance of the required particle size in the case of dispersions, and by the use of surfactants.

These compositions may also contain adjuvants such as preservatives, wetting agents, emulsifying agents, and dispersing agents. Prevention of the action of microorganisms may be ensured by the inclusion of various antibacterial and antifungal agents, for example, paraben, chlorobutanol, phenol sorbic acid, and the like. It may also be desirable to include isotonic agents such as sugars, sodium chloride, and the like. Prolonged absorption of the injectable pharmaceutical form may be brought about by the inclusion of agents that delay absorption such as aluminum monostearate and gelatin.

In some cases, in order to prolong the effect of the drug, it is desirable to slow the absorption of the drug from subcutaneous or intramuscular injection. This may be accomplished by the use of a liquid suspension of crystalline or amorphous material with poor water solubility. The rate of absorption of the drug then depends upon its rate of dissolution which, in turn, may depend upon crystal size and crystalline form. Alternatively, delayed absorption of a parenterally administered drug form is accomplished by dissolving or suspending the drug in an oil vehicle.

Injectable depot forms are made by forming microencapsule matrices of the drug in biodegradable polymers such as polylactide-polyglycolide. Depending upon the ratio of drug to polymer and the nature of the particular polymer employed, the rate of drug release can be controlled. Examples of other biodegradable polymers include poly(orthoesters) and poly(anhydrides). Depot injectable formulations are also prepared by entrapping the drug in liposomes or microemulsions which are compatible with body tissues.

The injectable formulations can be sterilized, for example, by filtration through a bacterial-retaining filter, or by incorporating sterilizing agents in the form of sterile solid compositions which can be dissolved or dispersed in sterile water or other sterile injectable medium just prior to use.

Solid dosage forms for oral administration include capsules, tablets, pills, powders, and granules. In such solid dosage forms, the active compound is mixed with at least one inert, pharmaceutically acceptable excipient or carrier such as sodium citrate or dicalcium phosphate and/or a) fillers or extenders such as starches, lactose, sucrose, glucose, mannitol, and silicic acid, b) binders such as, for example, carboxymethylcellulose, alginates, gelatin, polyvinylpyrrolidone, sucrose, and acacia, c) humectants such as glycerol, d) disintegrating agents such as agar-agar, calcium carbonate, potato or tapioca starch, alginic acid, certain silicates, and sodium carbonate, e) solution retarding agents such as paraffin, f) absorption accelerators such as quaternary ammonium compounds, g) wetting agents such as, for example, cetyl alcohol and glycerol monostearate, h) absorbents such as kaolin and bentonite clay, and i) lubricants such as talc, calcium stearate, magnesium stearate, solid polyethylene glycols, sodium lauryl sulfate, and mixtures thereof. In the case of capsules, tablets and pills, the dosage form may also comprise buffering agents.

Solid compositions of a similar type may also be employed as fillers in soft, semi-solid and hard-filled gelatin capsules or liquid-filled capsules using such excipients as lactose or milk sugar as well as high molecular weight polyethylene glycols and the like.

The solid dosage forms of tablets, dragees, capsules, pills, and granules can be prepared with coatings and shells such as enteric coatings and other coatings well known in the pharmaceutical formulating art. They may optionally contain opacifying agents and can also be of a composition that they release the active ingredient(s) only, or preferentially, in a certain part of the intestinal tract, optionally, in a delayed manner. Examples of embedding compositions that can be used include polymeric substances and waxes. Those embedding compositions containing a drug can be placed on medical devices, such as stents, grafts, catheters, and balloons.

The active compounds can also be in micro-encapsulated form, if appropriate, with one or more of the above-mentioned excipients.

Liquid dosage forms for oral administration include pharmaceutically acceptable emulsions, solutions, suspensions, syrups and elixirs. In addition to the active compounds, the liquid dosage forms may contain inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethyl formamide, oils (in particular, cottonseed, groundnut, corn, germ, olive, castor, and sesame oils), glycerol, tetrahydrofurfuryl alcohol, polyethylene glycols and fatty acid esters of sorbitan, and mixtures thereof.

Besides inert diluents, the oral compositions can also include adjuvants such as wetting agents, emulsifying and suspending agents, sweetening, flavoring, and perfuming agents.

Suspensions, in addition to the active compounds, may contain suspending agents as, for example, ethoxylated isostearyl alcohols, polyoxyethylene sorbitol and sorbitan esters, microcrystalline cellulose, aluminum metahydroxide, bentonite, agar-agar, and tragacanth, and mixtures thereof.

Topical administration includes administration to the skin or mucosa, including surfaces of the lung and eye. Compositions for topical administration, including those for inhalation, may be prepared as a dry powder which may be pressurized or non-pressurized. In non-pressurized powder compositions, the active ingredient in finely divided form may be used in admixture with a larger-sized pharmaceutically acceptable inert carrier comprising particles having a size, for example, of up to 100 micrometers in diameter. Suitable inert carriers include sugars such as lactose. Desirably, at least 95% by weight of the particles of the active ingredient have an effective particle size in the range of 0.01 to 10 micrometers. Compositions for topical use on the skin also include ointments, creams, lotions, and gels.

Alternatively, the composition may be pressurized and contain a compressed gas, such as nitrogen or a liquefied gas propellant. The liquefied propellant medium and indeed the total composition is preferably such that the active ingredient does not dissolve therein to any substantial extent. The pressurized composition may also contain a surface active agent. The surface active agent may be a liquid or solid non-ionic surface active agent or may be a solid anionic surface active agent. It is preferred to use the solid anionic surface active agent in the form of a sodium salt.

A further form of topical administration is to the eye, as for the treatment of immune-mediated conditions of the eye such as autoimmune diseases, allergic or inflammatory conditions, and corneal transplants. The compound of the invention is delivered in a pharmaceutically acceptable ophthalmic vehicle, such that the compound is maintained in contact with the ocular surface for a sufficient time period to allow the compound to penetrate the corneal and internal regions of the eye, as for example the anterior chamber, posterior chamber, vitreous body, aqueous humor, vitreous humor, cornea, iris/cilary, lens, choroid/retina and sclera. The pharmaceutically acceptable ophthalmic vehicle may, for example, be an ointment, vegetable oil or an encapsulating material.

Compositions for rectal or vaginal administration are preferably suppositories or retention enemas which can be prepared by mixing the compounds of this invention with suitable non-irritating excipients or carriers such as cocoa butter, polyethylene glycol or a suppository wax which are solid at room temperature but liquid at body temperature and therefore melt in the rectum or vaginal cavity and release the active compound.

Compounds of the present invention can also be administered in the form of liposomes. As is known in the art, liposomes are generally derived from phospholipids or other lipid substances. Liposomes are formed by mono- or multilamellar hydrated liquid crystals that are dispersed in an aqueous medium. Any non-toxic, physiologically acceptable and metabolizable lipid capable of forming liposomes can be used. The present compositions in liposome form can contain, in addition to a compound of the present invention, stabilizers, preservatives, excipients, and the like. The preferred lipids are the phospholipids and the phosphatidyl cholines (lecithins), both natural and synthetic. Methods to form liposomes are known in the art. See, for example, Prescott, Ed., Methods in Cell Biology, Volume XIV, Academic Press, New York, N.Y. (1976), p. 33 *et seq*.

Compounds of the present invention may also be coadministered with one or more immunosuppressant agents. The immunosuppressant agents within the scope of this invention include, but are not limited to, IMURAN^{®} azathioprine sodium, brequinar sodium, SPANIDIN^{®} gusperimus trihydrochloride (also known as deoxyspergualin), mizoribine (also known as bredinin), CELLCEPT^{®} mycophenolate mofetil, NEORAL^{®} Cylosporin A (also marketed as different formulation of Cyclosporin A under the trademark SANDIMMUNE^{®}), PROGRAF^{®} tacrolimus (also known as FK-506), sirolimus and RAPAMUNE^{®}, leflunomide (also known as HWA-486), glucocorticoids, such as prednisolone and its derivatives, antibody therapies such as orthoclone (OKT3) and Zenapax^{®}, and antithymyocyte globulins, such as thymoglobulins.

### Example 3

The purpose of this example was to determine the effects of a rapamycin analog on neointimal formation in porcine coronary arteries containing stents. This example illustrates that the rapamycin analog A-179578, when compounded and delivered from the Biocompatibles BiodiviYsio PC Coronary stent favorably affects neointimal hyperplasia and lumen size in porcine coronary arteries. This finding suggests that such a combination may be of substantial clinical benefit if properly applied in humans by limiting neointimal hyperplasia.

The agent A-179578 is a rapamycin analog. The study set forth in this example was designed to assess the ability of the rapamycin analog A-179578 to reduce neointimal hyperplasia in a porcine coronary stent model. Efficacy of A-179578 in this model would suggest its clinical potential for the limitation and treatment of coronary restenosis in stents following percutaneous revascularization. The domestic swine was used because this model appears to yield results comparable to other investigations seeking to limit neointimal hyperplasia in human subjects.

The example tested A-179578 eluted from coronary stents placed in juvenile farm pigs, and compared these results with control stents. The control stents had polymer alone covering its struts. This is important, for the polymer itself must not stimulate neointimal hyperplasia to a substantial degree. As the eluted drug disappears, an inflammatory response to the polymer could conceivably result in a late "catch-up phenomenon" where the restenosis process is not stopped, but instead slowed. This phenomenon would result in restenosis at late dates in human subjects.

Stents were implanted in two blood vessels in each pig. Pigs used in this model were generally 2-4 months old and weighed 30-40 Kg. Two coronary stents were thus implanted in each pig by visually assessing a Anormal@ stent:artery ratio of 1.1-1.2.

Beginning on the day of the procedure, pigs were given oral aspirin (325 mg daily) and continued for the remainder of their course. General anesthesia was achieved by means of intramuscular injection followed by intravenous ketamine (30 mg/kg) and xylazine (3 mg/kg). Additional medication at the time of induction included atropine (1 mg) and flocillin (1 g) administered intramuscularly, During the stenting procedure, an intraarterial bolus of 10,000 units of heparin was administered.

Arterial access was obtained by cutdown on the right external carotid and placement of an 8F sheath. After the procedure, the animals were maintained on a normal diet without cholesterol or other special supplementation.

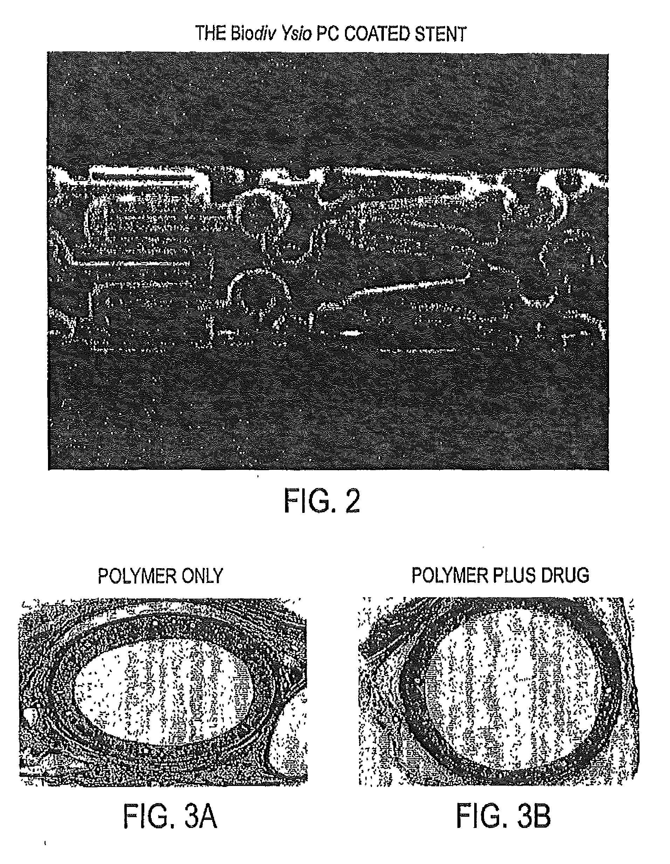

The BiodivYsio stent was used with nominal vessel target size of 3.0 mm. See Figure 2. Two coronary arteries per pig were assigned at random to deployment of the stents. The stent was either a drug eluting stent (polymer plus drug stent) or a stent coated with a polymer only (polymer only stent). The stents were delivered by means of standard guide catheters and wires. The stent balloons were inflated to appropriate sizes for less than 30 seconds.

Each pig had one polymer only stent and one polymer plus drug stent placed in separate coronary arteries, so that each pig would have one stent for drug and one for control.

A sample size of 20 pigs total was chosen to detect a projected difference in neointimal thickness of 0.2 mm with a standard deviation of 0.15 mm, at a power of 0.95 and beta 0.02.

Animals were euthanized at 28 days for histopathologic examination and quantification. Following removal of the heart from the perfusion pump system, the left atrial appendage was removed for access to the proximal coronary arteries. Coronary arterial segments with injuries were dissected free of the epicardium. Segments containing lesions was isolated, thereby allowing sufficient tissue to contain uninvolved blood vessel at either end. The foregoing segments, each roughly 2.5 cm in length, were embedded and processed by means of standard plastic embedding techniques. The tissues were subsequently processed and stained with hematoxylin-eosin and elastic-van Gieson techniques.

Low and high power light microscopy were used to make length measurements in the plane of microscopic view by means of a calibrated reticle and a digital microscopy system connected to a computer employing calibrated analysis software.

The severity of vessel injury and the neointimal response were measured by calibrated digital microscopy. The importance of the integrity of the internal elastic lamina is well-known to those skilled in the art. A histopathologic injury score in stented blood vessels has been validated as being closely related to neointimal thickness. This score is related to depth of injury and is as follows:

| Score | Description of Injury |
|---|---|
| 0 | Internal elastic lamina intact; endothelium typically denuded, media compressed but not lacerated. |
| 1 | Internal elastic lamina lacerated; media typically compressed but not lacerated. |
| 2 | Internal elastic lacerated; media visibly lacerated; external elastic lamina intact but compressed. |
| 3 | External elastic lamina lacerated; typically large lacerations of media extending through the external elastic lamina; coil wires sometimes residing in adventitia. |

This quantitative measurement of injury was assessed for all stent wires of each stent section. The calibrated digital image was also used to measure at each stent wire site the neointimal thickness. Lumen area, area contained with the internal elastic lamina, and area within the external elastic lamina were also measured.

At each stent wire site for a given section, the neointimal thickness was averaged to obtain a mean injury score for each section. The measurement of neointimal thickness was made to the abluminal side of the stent wire, because the neointimal in all cases includes this thickness.

The mid-stent segment was used for measurement, analysis, and comparison. Data were also recorded (and included in the data section of this report) for proximal and distal segments.

The data analysis methods for this study did not need to take into account variable arterial injury across treatment/control groups, because mild to moderate injury is sensitive enough to detect treatment differences. Paired t-testing was performed to compare variables across the polymer only stents (control group) and polymer plus drug stents (treatment group). No animal died in this study before scheduled timepoints.

Table 3 shows,the pigs and arteries used. In Table 3, LCX means the circumflex branch of the left coronary artery, LAD means the left anterior descending coronary artery, and RCA means the right coronary artery.

**Table 3**

| Pigs and Vessels Used | | |
|---|---|---|
| 1 | 2000-G-693 | RCA - Control |
| | 2000-G-693 | LCX - Test |
| 2 | 2000-G-698 | RCA - Test |
| | 2000-G-698 | LAD - Control |
| 3 | 2000-G-702 | RCA - Test |
| | 2000-G-702 | LAD - Control |
| 4 | 2000-G-709 | RCA - Control |
| | 2000-G-709 | LAD - Test |
| 5 | 2000-G-306 | RCA - Control |
| | 2000-G-306 | LAD - Test |
| | 2000-G-306 | * LCX - Test |
| 6 | 2000-G-672 | RCA - Test |
| | 2000-G-672 | LAD - Control |
| 7 | 2000-G-712 | RCA - Control |
| | 2000-G-712 | LCX - Test |
| 8 | 2000-G-735 | RCA - Control |
| | 2000-G-735 | LAD - Test |
| 9 | 2000-G-736 | CA - Control |
| | 2000-G-736 | LCX - Test |
| 10 | 2000-G-740 | RCA - Test |
| | 2000-G-740 | LAD - Control |
| 11 | 2000-G-742 | LAD - Test |
| | 2000-G-742 | OM ( LCX ) - Control |
| 12 | 2000-G-744 | RCA - Test |
| | 2000-G-744 | LAD - Control |
| 13 | 2000-G-748 | RCA - Test |
| | 2000-G-748 | LAD - Control |
| 14 | 2000-G-749 | RCA - Control |
| | 2000-G-749 | LCX - Test |
| 15 | 2000-G-753 | RCA - Control |
| | 2000-G-753 | LAD - Test |
| 16 | 2000-G-754 | RCA - Test |
| | 2000-G-754 | LCX -Control |
| 17 | 2000-G-755 | RCA - Control |
| | 2000-G-755 | LAD - Test |
| 18 | 2000-G-756 | RCA - Test |
| | 2000-G-756 | LAD - Control |
| 19 | 2000-G-757 | LAD - Control |
| | 2000-G-757 | LCX - Test |
| 20 | 2000-G-760 | LAD - Test |
| | 2000-G-760 | LCX -Control |

Table 4 shows the summary results for all data for mean injury and neointimal thickness for each stent, including proximal, mid, and distal segments. Table 4 also shows lumen size, percent stenosis, and artery size as measured by the internal elastic laminae (IEL) and external elastic laminae (EEL).

**Table 4**

| Summary: All Measures (Distal, Mid, Proximal) | | | | | | | | | |
|---|---|---|---|---|---|---|---|---|---|
| ID | prox ref | dist ref | Lumen injury | IEL | EEL | mean | % stenosis | Neointimal area | NIT |
| Control | Distal | | | | | | | | |
| Mean | 4.46 | 3.96 | 4.88 | 7.66 | 9.00 | 0.22 | 36.10 | 2.79 | 0.41 |
| SD | 1.20 | 1.16 | 1.30 | 1.15 | 1.10 | 0.26 | 15.41 | 1.29 | 0.17 |
| | | | | | | | | | |

| Control | Mid | | | | | | | | |
|---|---|---|---|---|---|---|---|---|---|
| Mean | 4.46 | 3.96 | 4.94 | 7.71 | 9.08 | 0.08 | 36.23 | 2.77 | 0.38 |
| SD | 1.20 | 1.16 | 1.44 | 1.07 | 1.15 | 0.14 | 14.93 | 1.20 | 0.16 |
| | | | | | | | | | |
| | | | | | | | | | |

| Control | Proximal | | | | | | | | |
|---|---|---|---|---|---|---|---|---|---|
| Mean | 4.46 | 3.96 | 5.11 | 7.89 | 9.30 | 0.15 | 35.35 | 2.78 | 0.38 |
| SD | 1.20 | 1.16 | 1.38 | 1.33 | 1.42 | 0.22 | 11.94 | 1.04 | 0.12 |
| | | | | | | | | | |

| Test | Distal | | | | | | | | |
|---|---|---|---|---|---|---|---|---|---|
| Mean | 4.26 | 3.41 | 6.04 | 7.70 | 9.01 | 0.26 | 22.35 | 1.66 | 0.25 |
| SD | 1.26 | 0.96 | 1.55 | 1.49 | 1.47 | 0.43 | 8.58 | 0.58 | 0.06 |
| | | | | | | | | | |

| Test | Mid | | | | | | | | |
|---|---|---|---|---|---|---|---|---|---|
| Mean | 4.26 | 3.41 | 6.35 | 7.75 | 8.98 | 0.04 | 18.71 | 1.41 | 0.22 |
| SD | 1.26 | 0.96 | 1.29 | 1.18 | 1.31 | 0.07 | 5.68 | 0.33 | 0.05 |
| | | | | | | | | | |

| Test | Proximal | | | | | | | | |
|---|---|---|---|---|---|---|---|---|---|
| Mean | 2.56 | 2.15 | 3.31 | 4.06 | 4.66 | 0.19 | 16.79 | 1.29 | 0.18 |
| SD | 1.66 | 1.37 | 2.39 | 3.48 | 4.15 | 0.13 | 9.97 | 0.80 | 0.12 |

There was no statistically significant difference for neointimal area or thickness across proximal, mid, or distal segments within the test group (polymer plus drug stents) or control groups (polymer only stents). This observation is quite consistent with prior studies, and thus allows use of only the mid segment for statistical comparison of test devices (polymer plus drug stents) vs. control devices (polymer only stents).

Table 5 shows the statistical t-test comparisons across test groups and control groups. There was a statistically significant difference in neointimal thickness, neointimal area, lumen size, and percent lumen stenosis, the drug eluting stent being clearly favored. Conversely, there were no statistically significant differences between the test group (polymer plus drug stents) and the control group (polymer only stents) for mean injury score, external elastic laminae, or internal elastic laminae areas.

**Table 5**

| Statistical Comparison of Test vs. Control Parameters: Mid-Section Data | | | | | | | |
|---|---|---|---|---|---|---|---|
| t-test Statistics | | | | | | | |
| | | | | | | | |

| Parameter | Difference | t-test | DF | Std Error | Lower 95% | Upper 95% | p |
|---|---|---|---|---|---|---|---|
| Lumen | -1.17 | -2.28 | 38 | 0.52 | -2.21 | -0.13 | 0.029 |
| IEL | 0.03 | 0.088 | 38 | 0.36 | -0.71 | 0.78 | 0.93 |
| | | | | | | | |
| EEL | 0.2 | 0.499 | 38 | 0.39 | -0.599 | 0.99 | 0.62 |
| | | | | | | | |
| NI Thickness | 0.18 | 5.153 | 38 | 0.034 | 0.106 | 0.244 | <.0001 |
| | | | | | | | |
| NI Area | 1.21 | 3.62 | 38 | 0.33 | 0.53 | 1.88 | 0.0008 |
| | | | | | | | |
| Mean Injury | 0.038 | 1.137 | 38 | 0.033 | -0.02 | 0.106 | 0.26 |
| | | | | | | | |
| % Stenosis | 14.54 | 2.97 | 38 | 4.9 | 4.61 | 24.47 | 0.005 |

The reference arteries proximal and distal to the stented segments were observed, and quantitated. These vessels appeared normal in all cases, uninjured in both the control group (polymer only stents) and the test group (polymer plus drug stents). See Figures 3A and 3B. The data below show there were no statistically significant differences in size between the stents in the control group and the stents in the test group.

| | | |
|---|---|---|
| | Proximal Reference | Distal Reference |
| | Diameter (mm) | Diameter (mm) |

| Control | | |
|---|---|---|
| (mean ± SD) | 4.46 ± 1.20 | 3.96 ± 1.16 |
| | | |

| Test | | |
|---|---|---|
| (mean + SD) | 4.26 ± 1.26 | 3.41 ± 0.96 |

The data suggest that statistically significant differences exist, and these differences favor the stent that elutes A-179578. The stent of this invention results in lower neointimal area, lower neointimal thickness, and greater lumen area. There were no significant differences within the test group (polymer plus drug stents) and the control group (polymer only stents) for neointimal or injury parameters. There were no significant differences in artery sizes (including the stent) for the control group compared to the test group. These latter findings suggest no significant difference in the arterial remodeling characteristics of the polymeric coating containing the drug.

At most, mild inflammation was found on both the polymer plus drug stent and the polymer only stent. This finding suggests that the polymer exhibits satisfactory biocompatibility, even without drug loading. Other studies show that when drug has completely gone from the polymer, the polymer itself creates enough inflammation to cause neointima. This phenomenon may be responsible for the late Acatch-up@ phenomenon of clinical late restenosis. Because the polymer in this example did not cause inflammation in the coronary arteries, late problems related to the polymer after the drug is exhausted are unlikely.

In conclusion, a stent containing the compound A-179578 with a polymer showed a reduction in neointimal hyperplasia in the porcine model when placed in a coronary artery.

### Example 4

The purpose of this example is to determine the rate of release of the A-179578 drug from 316L Electropolished Stainless Steel Coupons coated with a biocompatible polymer containing phosphoryiclloline side groups.

Rubber septa from lids from HPLC vials were removed from the vials and placed into glass vials so that the "Teflon" side faced up. These septa served as supports for the test samples. The test samples were 316L stainless steel coupons that had been previously coated with a biocompatible polymer containing phosphorylcholine side groups (PC polymer). Coronary stents are commonly made of 316L stainless steel and can be coated with the PC polymer to provide a depot site for loading drugs. The coated coupons, which serve to simulate stents, were placed onto the septa. By using a glass Hamilton Syringe, a solution of A-179578 and ethanol (10 µl) was applied to the surface of each coupon. The solution contained A-179578 (30.6 mg) dissolved in 100% ethanol (3.0 ml). The syringe was cleaned with ethanol between each application. The cap to the glass vial was placed on the vial loosely, thereby assuring proper ventilation. The coupon was allowed to dry for a minimum of 1.5 hours. Twelve (12) coupons were loaded in this way - six being used to determine the average amount of drug loaded onto the device and six being used to measure the time needed to release the drug from the devices.

To determine the total amount of A-179578 loaded onto a coupon, a coupon was removed from the vial and placed into 50/50 acetonitrile/ 0.01M phosphate buffer (pH 6.0, 5.0 ml). The coupon was placed onto a 5210 Branson sonicator for one hour. The coupon was then removed from the solution, and the solution was assayed by HPLC.

The time release studies were performed by immersing and removing the individual coupons from fresh aliquots (10.0 ml) of 0.01 M phosphate buffer at a pH of 6.0 at each of the following time intervals - 5, 15, 30 and 60 minutes. For the remaining time points of 120, 180, 240, 300, 360 minutes, volumes of 5.0 ml of buffer were used. To facilitate mixing during the drug release phase, the samples were placed onto an Eberbach shaker set at low speed. All solution aliquots were assayed by HPLC after the testing of the last sample was completed.

The HPLC analysis was performed with a Hewlett Packard series 1100 instrument having the following settings:

| | | |
|---|---|---|
| Injection Volume | = | 100 µl |
| Acquisition Time | = | 40 minutes |
| Flow Rate | = | 1.0 ml/min |
| Column Temperature | = | 40 °C |
| Wavelength | = | 278 nm |
| Mobile Phase | = | 65% Acetonitrile/35% H₂O |
| Column | = | YMC ODS-A S5 µm,4.6 x 250mm Part No. A12052546WT |

The results from the above experiment showed the following release data:

**Table 6**

| Time (min.) | Percent Release | Standard Deviation |
|---|---|---|
| 0.00 | 0.00 | 0.00 |
| 5.00 | 1.87 | 1.12 |
| 15.00 | 2.97 | 1.47 |
| 30.00 | 3.24 | 1.28 |
| 60.00 | 3.29 | 1.29 |
| 120.00 | 3.92 | 1.28 |
| 180.00 | 4.36 | 1.33 |
| 240.00 | 4.37 | 1.35 |
| 300.00 | 6.34 | 2.07 |
| 360.00 | 7.88 | 1.01 |

### Example 5

The purpose of this example was to determine the loading and release of A-179578 from 15mm BiodivYsio drug delivery stents.

To load the stents with drug, a solution of A-179578 in ethanol at a concentration of 50 mg/ml was prepared and dispensed into twelve vials, Twelve individual polymer-coated stents were placed on fixtures designed to hold the stent in a vertical position and the stents were immersed vertically in the drug solution for five minutes. The stents and fixtures were removed from the vials and excess drug solution was blotted away by contacting the stents with an absorbent material. The stents were then allowed to dry in air for 30 minutes in an inverted vertical position.

The stents were removed from the fixtures, and each stent was placed into 50/50 acetonitrile/phosphate buffer (pH 5.1, 2.0 ml) and sonicated for one hour. The stents were removed from the solution and solutions were assayed for concentration of drug, which allowed calculation of the amount of drug originally on the stents. This method was independently shown to remove at least 95% of the drug from the sent coating. On average, the stents contained 60 micrograms of drug ± 20 micrograms.

The drug-loaded stents were placed on the fixtures and placed into 0.01 M phosphate buffer (pH = 6.0, 1.9 ml) in individual vials. These samples were placed onto a Eberbach shaker set at low speed to provide back-and-forth agitation. To avoid approaching drug saturation in the buffer, the stents were transferred periodically to fresh buffer vials at the following points: 15, 30,45, 60,120,135, 150, 165, 180, 240, 390 minutes. The dissolution buffer vials were assayed by HPLC for the drug concentration at the end of the drug release period studied. The data, represented as % cumulative release of the drug as a function of time, is shown in tabular form below:

**Table 7**

| Time (min) | % Cumulative Release of Drug |
|---|---|
| 15 | 0.3 |
| 30 | 1.1 |
| 45 | 2.1 |
| 60 | 3.2 |
| 120 | 4.3 |
| 135 | 5.9 |
| 150 | 6.3 |
| 165 | 6.8 |
| 180 | 7.4 |
| 240 | 10.8 |
| 390 | 13.2 |

### Example 6

The purpose of this example was to evaluate the safety and efficacy of different drug dosages on neointima formation. Drug was delivered from the BiodivYsio OC stent (15mm) coated with A-179578. In-stent neointima formation was measured at four time intervals - 3 days, 1 month, and 3 months - in the coronary arteries of adult miniature swine. Forty (40) animals were studied at each time interval (10 animals per dose). Each animal received one drug-coated stent and one control stent. The control stent contained no drug. Table 8 shows the dosing scheme for swine efficacy study.

**Table 8**

| | Dose group 1 (µg) | Dose group 2 2(µg) | Dose group 3 (µg) | Dose group 4 (µg) |
|---|---|---|---|---|
| A-179578 per stent | 15 | 45 | 150 | 400 |
| A-179578permm of stent | 1 | 3 | 10 | 27 |

Potential local tissue toxicity was assessed at all time intervals by examining histopathologic changes in the stented region, adjacent coronary segments, perivascular tissue, and subserved myocardium. The mortality, angiographic implant and restudy data, histomorphometry data, and stent site histopathology were studied

### Three-Day Group

Histopathology in combination with scanning electron microscopy provided information regarding the short-term response to the implanted stent. The responses were similar in the control group and all dose groups, and the responses involved compression of the tunica media without remarkable necrosis, an accumulation of thrombus and inflammatory cells mostly localized to the stent struts, and early evidence of endothelial recovery and smooth muscle cell invasion of the thin mural thrombi. There were no extensive thrombi or remarkable intramural hemorrhages. The adventitia in some samples displayed either focal or diffuse inflammatory infiltrates, and occasionally, there was plugging or congestion of the vasa vasora. There was no evidence of medial necrosis in any sample.

Scanning electron microscopy showed similar appearance of the luminal surface three days after the implant of the coronary stent in all dose groups, The shape of the stent was clearly embedded in a thin layer of tissue. The endothelium was intact between the struts and even over the struts; a confluent or nearly confluent layer of endothelial-like cells had covered the luminal surface. There were scattered adherent platelets, platelet microthrombi, and leukocytes over the stents and on the intact remnant endothelium in the inter-strut spaces, In arteries with more severe stent-induced vessel damage, there were more substantial mural thrombi, but the extent of endothelial recovery over the stent struts did not appear retarded, regardless of the dosage of A-179578.

### One-Month Group

The histomorphometry data for the one-month series indicated a significant inhibitory effect of locally eluted A-179578 on neointima formation in stented coronary arteries of swine, Intima area normalized to injury score was significantly decreased for dose groups 3 and 4 (10 and 27 µg/mm) as compared with the control; there were also trends for decreases in absolute intima area and intima thickness for both dose groups 3 and 4 as compared with the control, and a tendency towards decreased histologic % stenosis for dose group 3 as compared with the control.

The control stents displayed morphology typical of stents implanted in coronary arteries of Yucatan miniature swine at one month. The tunica media was compressed or thinned without necrosis subjacent to profiles of stent struts; there were only occasional inflammatory infiltrates; and the neointima ranged in size from relatively thin to moderately thin, and were composed of spindle-shaped and stellate cells in an abundant extracellular matrix, with only rare small foci of fibrinoid material around the profiles of the stent struts. The drug-coated stents showed similar compression of the tunica media without any substantial necrosis at any dose; like control devices, there was little inflammation present. The neointima was notably thinner in dose groups 3 and 4, in some cases being composed of only a few,layers of cells. In all dose groups, there were substantial numbers of samples in which moderately sized fibrinoid deposits and inspisated thrombi were observed in the deep neointima. These were usually associated with the stent struts but sometimes extended between strut profiles. However, in no case was there exposure of thrombus on the luminal surface, as the deposits were encapsulated within fibrocellular tissue and covered with a flattened layer of periluminal endothelial-like cells.

Scanning electron microscopy confirmed that a confluent layer of endothelial or endothelial-like cells covered the entire stented surface, and there was no difference between drug-coated stents and control stents in terms of adherence of blood elements; leukocytes were present in approximately equal numbers in all groups. These findings demonstrate that while A-179578 was associated with decreased neointima formation and persistent mural thrombi, sufficient vessel wall healing in response to stent injury had occurred within one month after the stent had been implanted. This vessel wall healing had rendered the luminal surface nonreactive for platelet adhesion and thrombus formation, and minimally reactive for leukocyte adherence. Additionally, there was no evidence of vessel wall toxicity even at the highest dose (27µg/mm), as there was no medial necrosis or stent malapposition.

### Three-Month Group

There were no significant differences between the dose groups for any histomorphometric parameters of stented coronary arterial dimension in the three-month period of the study. However, there were weak trends for decreases in the two primary variables describing neointima formation - the cross-sectional area and the % area stenosis of the lumen.

The histopathologic appearance of the control stents in the swine coronary artery samples at three months after the implant appeared similar to that of the controls from the one-month group, and similar to those of all the groups in the three-month period. All samples showed fibrocellular neointima formation with mostly spindle-shaped smooth muscle-like cells in the neointima and a confluent squamous periluminal cell layer. There were no intramural hemorrhages or persistent fibrinoid deposits in the neointima; however some samples, particularly those with thicker neointima, showed evidence of prior thrombus accumulation and subsequent organization in the form of neovascularization in the neointima. On occasion, samples showed evidence of moderate to severe inflammatory reactions localized to the stent struts, associated with destruction of the tunica media architecture. These were most often associated with thicker neointima as well. However, these were few in number and were found in the control group as well as in the drug-coated stent groups. It is presumed that these represented either animal-specific generalized reactions to the implanted stent, evidence of contamination of the stent, or some combination of these two factors, and is commonly found at an incidence of about 10-15% in the studies of stent implants in swine coronary arteries. There was no evidence of necrosis of the tunica media or separation of the media from the stent in any sample. The adventitia of most three-month implants appeared to have somewhat greater neovascularization than did the one-month implants, but this did not appear related to control or test stent group. Scanning electron microscopy demonstrated confluent endothelium with rare adherent blood cells in the control group and all dose groups.

### Conclusions

The stent coated with A-179578 reduced in-stent neointima formation in swine coronary arteries and provided clear evidence of a biologic drug effect (unresorbed thrombus/fibrin deposits of neointima) at one month. There was a weak tendency for the stent coated with A-179578 to show a persistent inhibitory effect at the longerterm time interval of three months. There was no local coronary arterial wall toxicity in the form of medial necrosis or stent malapposition associated with any dose group, including the highest dose of approximately 27 µg/mm stent length at any time interval examined. All stents were well incorporated into the tissue, and there was evidence of stable healing responses in the form of fibrocellular neointimal incorporation and endothelial coverage at the one-month interval and at the three-month interval. The trend towards a sustained inhibitory effect at three months after the stent was implanted in this animal is surprising and provides evidence for potentially persistent effects in preventing clinical restenosis resulting from implanted stents.

Another embodiment of the present invention relates to a medical device comprising a coating containing at least one beneficial agent, such as a drug, and a hydration inhibitor that reduces the delivery rate of the beneficial agent. The coating may also contain other beneficial agents (e.g., another drug), a polymer or other substances (e.g., nonbiologically active substances) added to facilitate drug loading or other purposes.

In a preferred embodiment, the medical device is a drug eluting stent having a drug combination (i.e., more than one beneficial agent) in which a relatively less hydrophilic drug alters or controls the release of a relatively more hydrophilic drug. In this embodiment, the relatively less hydrophilic drug acts as the hydration inhibitor. The relatively less hydrophilic drug reduces the delivery or elution rate, e.g., by encapsulating, reducing hydration or otherwise interfering with diffusion, dissolution, permeation or other transport mechanisms, of the relatively more hydrophilic drug.

The phrases "relatively less hydrophilic" and "relatively more hydrophilic" as used herein relate to partitioning in water compared to partitioning in another substance. As described in CRC Handbook of Chemistry and Physics (3rd Electronic Edition) 2000, the octanol-water partition coefficient, P, is a widely used parameter for correlating biological effects of organic substances. It is a property of the two-phase system in which water and 1-octanol are in equilibrium at a fixed temperature and the substance is distributed between the water-rich and octanol-rich phases. P is defined as the ratio of the equilibrium concentration of the substance in the octanol-rich phase to that in the water-rich phase, in the limit of zero concentration. In general, P tends to be large for compounds with extended nonpolar structures (such as long chain or multiring hydrocarbons) and small for compounds with highly polar groups. Thus P (or, in its more common form of expression, log P) provides a measure of the lipophilic vs. hydrophilic nature of a compound, which is an important consideration in assessing the potential toxicity. A discussion of methods of measurement and accuracy considerations for log P is found in Sangster, J., J. Phys. Chem. Ref. Data, 18, 1111, 1989, incorporated herein by reference. LogP values can also be calculated by the method described in Hansch C. and Leo A. "Substituent Constants for Correlation Analysis in Chemistry and Biology" Wiley, N.Y., 1979. Other discussions of LogP may be found in the following documents, incorporated herein by reference: Mackay, D., Shiu, W.Y., and Ma, K.C., Illustrated Handbook of Physical-Chemical Properties and Environmental Fate for Organic Chemicals, Lewis Publishers/CRC Press, Boca Raton, FL, 1992; Shiu, W.Y., and Mackay, D., J. Phys. Chem. Ref. Data, 15, 911, 1986; Pinsuwan, S., Li, L., and Yalkowsky, S.H., J. Chem. Eng. Data, 40, 623, 995; Solubility Data Series, International Union of Pure and Applied Chemistry, Vol. 20, Pergamon Press, Oxford, 1985; Solubility Data Series, International Union of Pure and Applied Chemistry, Vol. 38, Pergamon Press, Oxford, 1985; Miller, M.M., Ghodbane, S., Wasik, S.P., Tewari, Y.B., and Martire, D.E., J. Chem. Eng. Data, 29, 184, 1984.

The LogP value of the beneficial agent should be less than the LogP value of the hydration inhibitor, as explained below. Preferably, the LogP value of the beneficial agent is less than 4.5 units and more preferably less than 3.0 units. However, it is possible for a compound to serve as a hydration inhibitor of the elution of a given compound according to the present invention when its LogP value is at least 0.5 units less than that of the given compound.

Although those skilled in the art are familiar with LogP values and the well-known methods for calculation thereof, Table 9 provides LogP values for several drugs.

**Table. 9 Log P Values**

| | |
|---|---|
| Probucol | >8 |
| Linolenic acid | >6 |
| Linoleic acid | >6 |
| Stearic acid | >6 |
| Oleic acid | >6 |
| Paclitaxel | >5 |
| Danazol | 4.5 |
| Rapamycin | >4.5 |
| ABT-578 | >4.5 |
| Tacrolimus | >4.5 |
| Fenofibrate | >4.5 |
| Indomethacin | 4.3 |
| Phenyl salicylate | 4.1 |
| B-estradiol | 4 |
| Vinblastine | 3.6 |
| ABT-627 | 3.4 |
| Testosterone | 3.3 |
| Progesterone | 3.2 |
| Paclitaxel | >3 |
| Cyclosporin A | 2.9 |
| Vincristine | 2.6 |
| Carvedilol | 1.97 |
| Dexamethasone | ∼1.9-2.2 |
| Vindesine | 1.3 |
| Dipyridamole | 1-2 |
| Methotrexate | -1.85 |

Those skilled in the art can calculate the Log P values for other compounds using techniques well known in the art.

Medical devices suitable for use in the present invention include, but are not limited to, interventional components such as coronary stents, peripheral stents, catheters, arterio-venous stents, by-pass grafts, drug delivery balloons used in the vasculature, filters, coils, staples, sutures, guidewires, catheters and catheter balloons. The devices are suitably composed of metal or polymeric materials, though other materials may be used. The phrase "interventional component" is used interchangeably herein with the term "prosthesis."

Medical devices to which coatings are applied according to the invention can be pretreated to prepare the surfaces for application of coatings. For example, stainless steel stents may be electropolished prior to coating (e.g., undercoat) application. Useful medical devices can be formed from NITINOL alloy, TRIPLEX (stainless steel/tantalum/stainless steel layer) or cobalt chromium alloy.

The interventional component may include at least one reservoir or cavity therein for retaining and supplying compounds in accordance with the present invention. In accordance with another aspect of the invention, one or more of the reservoirs or cavities is loaded with a more hydrophilic first beneficial agent and then a second more hydrophobic beneficial agent may be loaded onto the first beneficial agent within the cavity or reservoir in a manner as described above.

"Beneficial agent" as used herein, refers to any compound, mixture of compounds, or composition of matter consisting of a compound, which produces a beneficial or useful result. The beneficial agent may be a marker, such as a radiopaque dye or particles, or may be a drug, including pharmaceutical and therapeutic agents, or an agent including inorganic or organic drugs without limitation. The agent or drug can be in various forms such as uncharged molecules, components of molecular complexes, pharmacologically acceptable salts such as hydrochloride, hydrobromide, sulfate, laurate, paimitate, phosphate, nitrate, borate, acetate, maleate, tartrate, oleate, and salicylate.

Beneficial agents according to the invention also include antithrombotics, anticoagulants, antiplatelet agents, anti-lipid agents, thrombolytics, thrombospondin mimetics, antiproliferatives, anti-inflammatories, agents that inhibit hyperplasia, smooth muscle cell inhibitors, antibiotics, growth factor inhibitors, cell adhesion inhibitors, cell adhesion promoters, antimitotics, antifibrins, antioxidants, antineoplastics, agents that promote endothelial cell recovery, antiallergic substances, viral vectors, nucleic acids, monoclonal antibodies, antisense compounds, oligonucleotides, cell permeation enhancers, tissue permeation enhancers, and pro-drugs thereof, and hydrophilic radio markers including metrizamide, iopamidol, iohexol, iopromide, iobitriol, iomeprol, iopentol, ioversol, ioxilan, iodixanol, iotrolan, and analogs, derivatives and combinations thereof.

Examples of such antithrombotics, anticoagulants, antiplatelet agents, thrombolytics include sodium heparin, low molecular weight heparins, heparinoids, hirudin, argatroban, forskolin, vapriprost, prostacyclin and prostacylin analogues, dextran, D-phe-pro-arg-chloromethylketone (synthetic antithrombin), dipyridamole, glycoprotein IIbIllla (platelet membrane receptor antagonist antibody), recombinant hirudin, and thrombin inhibitors such as Angiomax™, from Biogen, Inc., Cambridge, Mass; thrombolytic agents e.g., urokinase Abbokinase™ from Abbott Laboratories Inc., North Chicago, IL, recombinant urokinase and pro-urokinase from Abbott Laboratories Inc., tissue plasminogen activator (Alteplase™ from Genentech, South San Francisco, CA and tenecteplase (TNK-tPA).

Examples of such cytostatic or antiproliferative agents include rapamycin and its analogs (such as everolimus, ABT-578-3S,6R,7E,9R,10R,12R,14S,15E,17E,19E,21S,23S,26R,27R,34aS)-9,10,12,13,14,21,22,23,24,25,26,27,32,33,34,34a-Hexadecahydro-9,27-dihydroxy-3-[(1R)-2-[(1S,3R,4R)-3-methoxy-4-tetrazol-1-yl)cyclohexyl]-1-methylethyl]-10,21-dimethoxy-6,8,12,14,20,26-hexamethyl-23,27-epoxy-3H-pyrido[2,1-c][1,4]oxaazacyclohentriacontine-1,5,11,28,29(4H,6H,31H)-pentone;23,27-Epoxy-3H pyrido[2,1-c][1,4]oxaazacyclohentriacontine-1,5,11,28,29(4H,6H,31 H)-pentone, tacrolimus and pimecrolimus) angiopeptin, angiotensin converting enzyme inhibitors such as captopril, e.g, Capoten® and Capozide® from Bristol-Myers Squibb Co., Stamford, Conn., cilazapril or lisinopril, e.g., Prinivil® and Prinzide® from Merck & Co., Inc., Whitehouse Station, NJ; calcium channel blockers such as nifedipine, amlodipine, cilnidipine, lercanidipine, benidipine, trifluperazine, diltiazem and verapamil; fibroblast growth factor antagonists, fish oil (omega 3-fatty acid), histamine antagonists, iovastatin, e.g. Mevacor® from Merck & Co., Inc., Whitehouse Station, NJ. In addition, topoisomerase inhibitors such as etoposide and topotecan, as well as antiestrogens such as tamoxifen may be used for delivery by ink-jetting.

Examples of such anti-inflammatories include colchicine and glucocorticoids such as betamethasone, cortisone, dexamethasone, budesonide, prednisolone, methylprednisolone and hydrocortisone. Non-steroidal anti-inflammatory agents include flurbiprofen, ibuprofen, ketoprofen, fenoprofen, naproxen, diclofenac, diflunisal, acetominophen, indomethacin, sulindac, etodolac, diclofenac, ketorolac, meclofenamic acid, piroxicam and phenylbutazone.

Examples of such antineoplastics include alkylating agents such as altretamine, bendamucine, carboplatin, carmustine, cisplatin, cyclophosphamide, fotemustine, ifosfamide, lomustine, nimustine, prednimustine, and treosulfin, antimitotics such as vincristine, vinblastine, paclitaxel, e.g., TAXOL® by Bristol-Myers Squibb Co., Stamford, Conn., docetaxel, e.g., Taxotere® from Aventis S.A., Frankfort, Germany, antimetabolites such as methotrexate, mercaptopurine, pentostatin, trimetrexate, gemcitabine, azathioprine, and fluorouracil, and antibiotics such as doxorubicin hydrochloride, e.g., Adrlamycin® from Pharmacia & Upjohn, Peapack, NJ, and mitomycin, e.g., Mutamycin® from Bristol-Myers Squibb Co., Stamford, Conn, agents that promote endothelial cell recovery such as estradiol

Additional drugs which may be utilized in this application include inhibitors of tyrosine kinase such as RPR-101511A, PPAR-alpha agonists such as Tricor™ (fenofibrate) from Abbott Laboratories Inc., North Chicago, IL, endothelin receptor antagonists such as ABT-627 from Abbott Laboratories Inc., North Chicago, IL; matrix metalloproteinase inhibitors such as ABT-518 from Abbott Laboratories Inc., North Chicago, IL, antiallergic agents such as permirolast potassium nitroprusside, phosphodiesterase inhibitors, prostaglandin inhibitors, suramin, serotonin blockers, steroids, thioprotease inhibitors, triazolopyrimidine, and nitric oxide.

While the foregoing beneficial agents are well known for their preventive and treatment properties, the substances or agents are provided by way of example and are not meant to be limiting. Further, other beneficial agents that are currently available or may be developed are equally applicable for use with the present invention.

If desired or necessary, the beneficial agent may include a binder to carry load or allow sustained release of an agent, such as but not limited to a suitable polymer or similar carrier. The term "polymer" is intended to include a product of a polymerization reaction inclusive of homopolymers, copolymers, terpolymers, etc., whether natural or synthetic, including random, alternating, block, graft, branched, cross-linked, blends, compositions of blends and variations thereof. The polymer may be in true solution, saturated, or suspended as particles or supersaturated in the beneficial agent. The polymer may be biocompatible, or biodegradable. It is preferred that, where used, the amount of (hydrophobic) polymer is relatively small compared to the amount of beneficial agent according to the invention.

Beneficial agents to be delivered from the interventional components of the present invention are relatively more hydrophilic than the hydration inhibitor. These beneficial agents include dexamethasone, ABT-627 (also known as atrasentan, a available from Abbott Laboratories, North Chicago, II. and disclosed in US -2002-0055457 A1, the disclosure of which is incorporated by reference and having the following structure 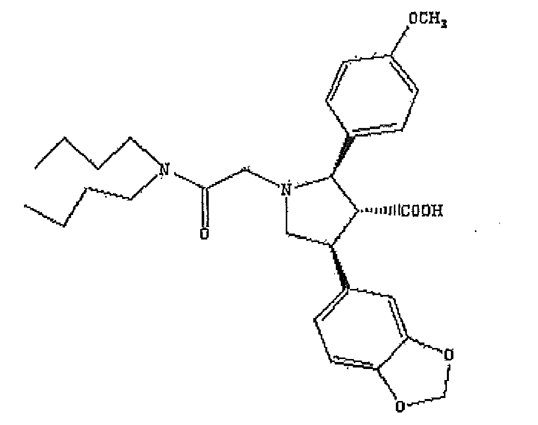 , dipyridamole, and estradiol. Preferred beneficial agents include dipyridamole, folic acid, estradiol, dexamethasone, and pro-drugs, analogs, derivatives, or combinations thereof

In accordance with some aspects of the invention, an interventional component can be loaded with first and second beneficial agents wherein one of the beneficial agents is more hydrophobic than the other. In this manner, the more hydrophobic beneficial agent acts as a water barrier for the less hydrophobic beneficial agent, thereby reducing the release rate of the less hydrophobic beneficial agent. For example and not limitation, the less hydrophobic beneficial agent may be ABT 620 {1-Methyl-N-(3,4,5-trimethoxyphenyl)-1 H-indole-5-sulfonamide), ABT' 627, ABT 518 {[S-(R*,R*)}-N-[1-(2,2-dimethyl-1,3-dioxol-4-yl)-2-[[4-[4-(trifluoro-methoxy)-phenoxy]phenyl]sulfonyl]ethyl]-N-hydroxyformamide }, dexamethasone and the like and the more hydrophobic beneficial agent may be fenofibrate, Tricor™ or 35,6R,7E,9R,10R,12R,14S,15E,17E,19E,21S,235,26R,27R,34aS)-9,10,12,13,14,21.22,23,24,25,26,27,32,33,34,34a-Hexadecahydro-9,27-dihydroxy-3-[(1R)-2-[(1S,3R,4R)-3-methoxy-4-tetrazol-1-yl)cyclohexyl]-1-methylethyl]-10,21-dimethoxy-6,8,12,14,20,26-hexamethyl-23,27-epoxy-3H-pyrido[2,1-c][1,4]oxaazacyclohentriacontine-1,5,11,28,29(4H,6H,31H)-pentone; 23,27-Epoxy-3H-pyrido[2,1-c][1,4]oxaazacyclohentriaconfine-1,5,11,28,29(4H,6H,31H)-pentone.

Devices according to the invention can include more than one beneficial agent. In these embodiments, the beneficial agents and hydration inhibitor can be selected such that the beneficial agents are delivered substantially simultaneously or in a preferred order, depending on the medical conditions to be treated. For example, delivery of a first beneficial agent may be alternated with delivery of a second beneficial agent. Or one beneficial agent may have a substantially constant delivery rate while delivery of a second beneficial agent is delayed. This staged delivery is one means according to the invention of controlling burst effect, commonly observed when a second drug is relatively hydrophilic and is hydrated immediately during implantation and/or positioning in the body. It is also possible to delivery a first therapeutic agent for a period of time before a second beneficial agent is delivered. Those skilled in the art will appreciate that these concepts can be expanded to three or more beneficial agents, as exemplified below.

An effective amount of hydration inhibitor according to the invention is that amount that shifts the contact angle of a mixture of beneficial agent and hydration inhibitor to at least 50 degrees, and more preferably 70 degrees. Contact angles and the methods for calculation are well known and are discussed, for example, in greater detail in McGraw-Hill Encyclopedia of Chemistry, Second Edition 1993, page 538, Sybil P. Parker, Editor in Chief, incorporated herein by reference.

Suitable hydration inhibitors can also include polymeric materials, markers or other additives. For example, hydration inhibitors may be relatively hydrophobic nonbiologically active substances. These substances include nitrophenyl octyl ether, bisethylhexyl sebacate and diisododecylphthalate and other hydrophobic compounds cited in U.S. 4 476 007, incorporated herein by reference, long chain alkanes, e.g., dodecane, butylated hydroxytolueno, butylated hydroxyanisole, or other antioxidants, diatrizoates, iothalamates, metrizoates, including amidotrizoic acid, iotalamic acid, adipiodone, ioxaglic acid, ethiodized oil.

In a preferred embodiment, the hydration inhibitor is a drug. Thus, medical devices according to the invention can deliver multiple beneficial agents. Drugs that are suitable for use in the present invention as a hydration inhibitor include, but are not limited to, ABT-578-- a tetrazole derivative of rapamycin having the following structure and a proprietary compound of Abbott Laboratories (U.S. Patent Nos. 6010815 and 6329386, incorporated herein by reference) 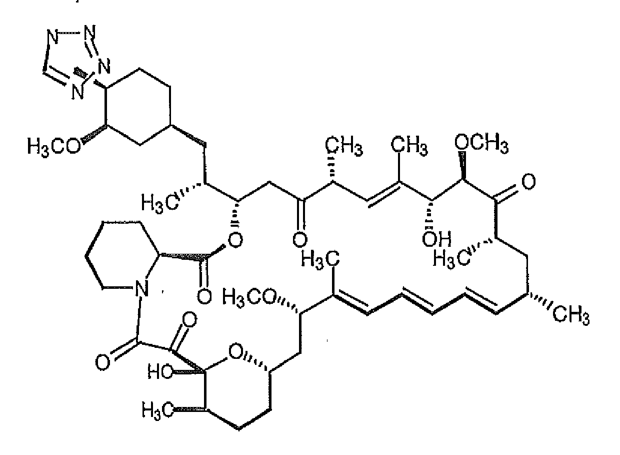 paclitaxel, rapamycin, rapamycin analogs and derivatives, including pimecrolimus and everolimus, fenofibrate, carvedilol, taxoteres, beta-carotene, tocopherols, tocotrienols, and tacrolimus. As indicated above, selection of suitable drugs that can be a hydration inhibitor in the present invention is based on the relative values of LogP for the hydration inhibitor and the beneficial agent to be delivered.

The coatings optionally include a polymeric material, e.g., phosphorylcholine, polycaprolactone, poly-D,L-lactic acid, poly-L-lactic acid, poly(lactide-co-glycolide), poly(hydroxybutyrate), poly(hydroxybutyrate-co-valerate), polydioxanone, polyorthoester, polyanhydride, poly(glycolic acid), poly(glycolic acid-co-trimethylene carbonate), polyphosphoester, polyphosphoester urethane, poly(amino acids), cyanoacrylates, poly(trimethylene carbonate), poly(iminocarbonate), polyalkylene oxalates, polyphosphazenes, polyiminocarbonates, and aliphatic polycarbonates, fibrin, fibrinogen, cellulose, starch, collagen, Parylene® brand poly-para-xylylene (available from SCSCookson Industries, Indianapolis, Indiana), Paryl AST™ brand biocompatible dielectric polymer (U.S. Patent Nos. 5,355,832 and 5,447,799, commercially available from AST Products of Billerica, MA) , polyurethane, polycarbonate urethanes, polyethylene, polyethylene terephthalate, ethylene vinyl acetate, ethylene vinyl alcohol, silicone polysiloxanes, substituted polysiloxanes, polyethylene oxide, polybutylene terephthalate-co-PEG, PCL-co-PEG (i.e., polycaprolactone co-polyethylene glycol), PLA-co-PEG (i.e., polylactic acid-co-polyethylene glycol), polyacrylates, polyvinyl pyrrolidone, polyacrylamide, thermoplastic elastomers, polyolefin elastomers, EPDM rubbers, polyamide elastomers, blostable plastic, acrylic polymers, nylon, polyesters, epoxies and derivatives or blends thereof (e.g., PLLA-phosphorylcholine).

In any of the embodiments disclosed herein, a porous or biodegradable membrane or layer made of biocompatible materials may be coated over the beneficial agent for sustained release thereof, if desired. Alternatively, a suitable base coating capable of retaining beneficial agent therein can be applied uniformly over the surface of the prosthesis, and then selected portions of the base coating can be loaded with the beneficial agent in accordance with the invention. A greater amount of beneficial agent can be loaded over a unit surface area intended to have a greater local areal density and a lower amount of beneficial agent can be loaded over a unit surface area intended to have a lower local areal density.

In yet another embodiment of the present invention, the beneficial agent may be applied directly to the surface of the prosthesis. Generally a binder or similar component may be used to ensure sufficient adhesion. For example, this coating technique may include admixing the beneficial agent with a suitable binder or polymer to form a coating mixture, which is then coated onto the surface of the prosthesis. The coating mixture would be prepared in higher or lower concentrations of beneficial agent as desired, and then applied to selected portions of the prosthesis appropriately.

As noted above, the beneficial agent may be applied to the interventional component in a polymer, such as drug/polymer mixture. Preferably, the amount of polymer in the mixture is small compared to the amount of drug. For example, the polymer can be about 10% of the amount of drug. In these embodiments, the polymer facilitates processing or loading or enhances retention of the drug on the interventional device, but is in an amount that is not effective to substantially inhibit the hydration of the drug. The presence of the hydration inhibitor of suitable LogP as set forth above has the greater influence on delivery of the drug in this circumstance.

In accordance with some embodiments of the invention, the first and second beneficial agents may correspond to drug-polymer mixtures having different concentrations of polymer to effect different release rates of the particular drug in each beneficial agent. For example, the drug-polymer mixture having a higher concentration of polymer would have a slower release of the drug within the lumen. In contrast, the drug-polymer mixture having a lower concentration of polymer would cause a more rapid release of the drug. Alternatively, rather than providing drug-polymer mixtures having different polymer concentrations to provide different release rates, it is also possible to dispense beneficial agents within different polymers or other binders, wherein the specific polymer or binder has different diffusivity or affinity to assure delivery of the beneficial agents at different rates. Thus, in accordance with the invention, multiple beneficial agents can be released at rates appropriate for their activities and the prosthesis of the invention has multiple beneficial agents that elute off the prosthesis at desired rates.

For example, a cationic phosphorylcholine which has a higher affinity for anionic therapeutic agents can be blended and dispersed as a first beneficial agent and lipophilic phosphorylcholine can be blended with lipophilic drugs as the second beneficial agent to effect different release rates respectively.

As discussed in greater detail below, the beneficial agent(s) and hydration inhibitors can be applied to the medical device in one or more coating layers. For example, alternating layers may be used to control delivery of multiple beneficial agents. Beneficial agents can be applied to the medical device alone or in combination with a suitable hydration inhibitor. Coatings that are suitable for use in this invention include, but are not limited to, any biocompatible polymeric material having suitable mechanical properties and in which the beneficial agent(s) is substantially soluble.

Conventional coating techniques also may be utilized to coat the beneficial agent onto the surface of the prosthesis such as spraying, dipping or sputtering and still provide the desired effect if performed appropriately. With such techniques, it may be desirable or necessary to use known masking or extraction techniques to control the location and amount in which beneficial agent is loaded.

According to some embodiments of the present invention, the beneficial agent may be loaded directly onto a component (e.g., by pipetting) or alternatively, the beneficial agent is loaded onto a base material layer that is applied a surface of the component (e.g., dip loading). For example and not limitation, a base coating, such as a binder or suitable polymer, is applied to a selected surface of the interventional component. If desired, a pattern may be formed on a component surface. Beneficial agent is then applied directly to the pattern of the base material.

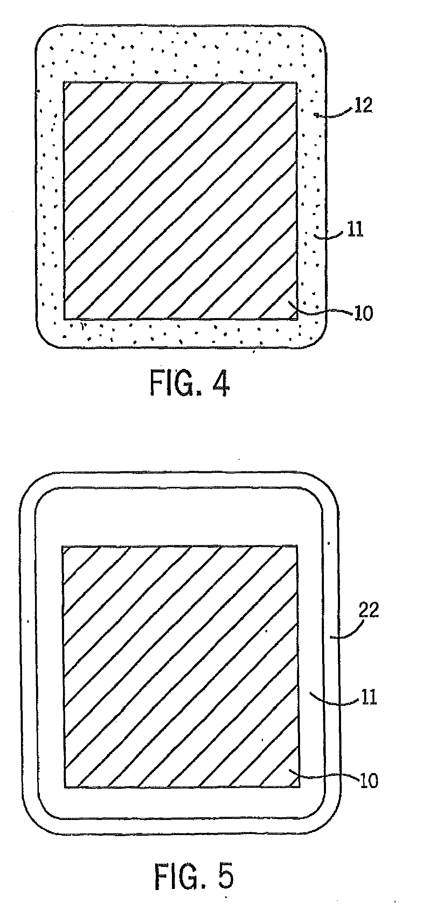

Figures 4-7 exemplify these various embodiments.

Figure 4 shows a cross-sectional view of a stent strut 10 having a layer of a beneficial agent 11 and hydration inhibitor 12 in a mixture. The mixture may be applied directly to the stent surface. Hydration inhibitor is present in an effective amount to control delivery of beneficial agent from the stent.

Figure 5 shows a cross-sectional view of a stent strut 10 having a first layer of a beneficial agent 11 and a second layer of a second beneficial agent 22 acting as a hydration inhibitor. According to this embodiment, delivery of the first beneficial agent is controlled by the second beneficial agent, especially where the second beneficial layer is not substantially polymeric, i.e., the amount of polymer is relatively small compared to the first beneficial agent.

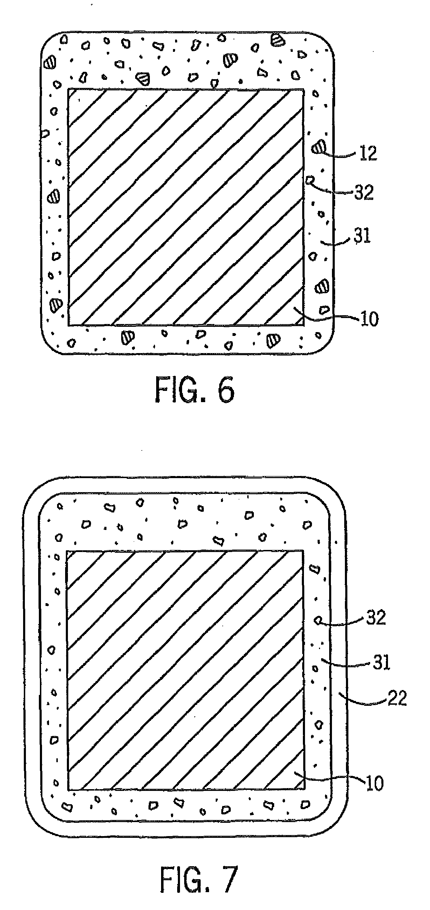

Figure 6 is a cross-sectional view of a stent strut 10 having a base layer of polymer material 31 loaded with a mixture of a beneficial agent 32 and a hydration inhibitor 12. Beneficial agent and hydration inhibitor are associated with each other as a mixture created and then loaded in a polymer. Release of beneficial agent is controlled by the presence of hydration inhibitor.

Figure 7 is a cross-sectional view of a stent strut 10 having a base layer of a polymer material 31 loaded with a first beneficial agent 32 and a layer of a second beneficial agent 22 acting as a hydration inhibitor. Release of the first beneficial agent in polymer is controlled by the overlying layer of the second beneficial agent.

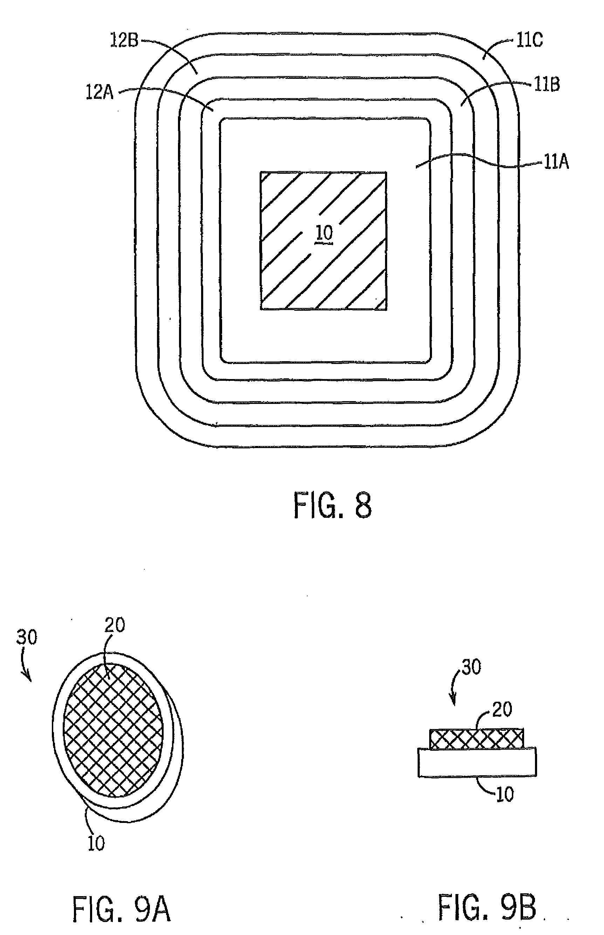

Figure 8 is a cross sectional view of a stent strut 10 having layers 11A, 11 B and 11 C of a first beneficial agent alternating with layers 12A and 12B of a second beneficial agent/hydration inhibitor. According to this embodiment, first beneficial agent, e.g., estradiol, from layer 11C elutes in an initial burst. Second beneficial agent/hydration inhibitor, e.g., ABT-578, in layer 12B controls elution of first beneficial agent from layer 11 B. Thus, the LogP of the second beneficial agent/hydration inhibitor is greater than the LogP of the first beneficial agent, in accordance with principles of the present invention. Similarly, second beneficial agent/hydration inhibitor in layer 12A controls elution of first beneficial agent in layer 11A. Layers 12A and 12B enable midterm and late term delivery of first beneficial agent along with second beneficial agent/hydration inhibitor. Depending on the beneficial agents selected, layers 11A, 11B, 11C, 12A and 12B may optionally contain a polymer carrier or binder or other additive to facilitate processing or retention of the beneficial agent on the interventional device.

As those skilled in the art will appreciate, many variations of this embodiment are possible, depending on the medical condition(s) being treated, number and identity of beneficial agents selected, desired order of delivery and other factors. For example, layers 11 A, 11 B and 11C need not contain the same beneficial agent. Each can contain a different beneficial agent or two can contain the same beneficial agent with the third containing another beneficial agent. Similarly, layers 12A and 12B need not contain the same beneficial agent. Although not shown here, even more complicated variations can be achieved by those skilled in the art using the principles disclosed herein. For example, it may be desirable to achieve a relatively early delivery of estradiol to treat surface monocytes and a delayed delivery of dexamethasone to treat tissue monocytes and macrophages.

The following example sets forth a technique for making the medical devices according to the present invention. Those skilled in the art will appreciate that other techniques for preparing medical devices according to the invention are available. Although some examples set forth medical devices having coating layers that include polymers, these polymers are optional. Medical devices in which the beneficial agents are not applied using a polymer are still within the scope of the present invention.

### Example 7

### 1. Coating the Coupon with PC1036

Prior to any experimentation, coated stainless steel coupons were prepared. These coupons were 316L electropolished stainless steel discs (10 mm diameter). This size was chosen because the surface area of one side of the coupon is similar to the surface area of a 15-mm open cell BiodivYsio stent. The coupon was prepared by scratching a mark on one side of the coupon, to indicate the side of the coupon that will not be coated, and then cleaned. The cleaning was a two-step process in which the coupons are sonicated for 3 minutes in dichloromethylene and 3 minutes in ethanol. The coupons were allowed to dry at room temperature. One side of the coupon was coated using a filtered 20-mg/mL solution of phosphoryl choline polymer PC1036 (product of Biocompatibles, Surrey, UK) in ethanol. Twenty µL PC solution was placed onto the coupon using a gas tight glass syringe, ensuring that the entire surface was coated but not spilling over the sides of the coupon. The coupons were initially air dried and then cured at 70°C for 16 hours. They were then sent for gamma irradiation at <25KGy. The resulting PC coating (20) thickness was close to that of the coupon (10) and thick enough to accommodate the desired loaded drug dose, as graphically represented in Figures 9A and 9B showing top and side views, respectively, of a PC-coated coupon (30).

### 11. Loading the Coupon with Drugs of interest

In these experiments, beneficial agents were loaded onto coupons and elution profiles examined. In general, the procedure is as follows. Twelve PC-coated coupons were loaded with each drug. The solutions of the drugs were usually 5.0 mg/mL in 100% ethanol and were filtered with a 0.45 µm filter prior to use.

The coupons were weighed before loading with the drug solution. To load 100 µg of drug, 20 µL of solution was placed (e.g., pipetted) on the center of the PC coated side of the coupon. The coupon was placed in a vial for 30 minutes with the lid closed to allow the drug to penetrate the coating. The lid was removed and the coupon was allowed to dry for an additional 90 minutes. To ensure that the coupon was completely dry, the coupon was weighed, and after 15 minutes the coupon was weighed a third time. When two weightings of the coupon were the same, the coupon was considered dry. The loaded, dry coupons were stored in a refrigerator protected from light.

### III. Extracting Drugs from the Coupon

For each drug, six coupons were used to evaluate the total amount of drug loaded by the above procedure. The coupons were immersed in 5 mL of 50% ethanol, 50% water solution and sonicated for 1 hour. The concentration of the drug in the extraction solution was analyzed by HPLC.

At the end of the elution experiments discussed below, the coupons were removed from the elution media and immersed in 5 mL of 50% ethanol, 50% water solution and sonicated for 1 hour. The concentration of the drug in these vials indicated the amount of the drug remaining in the coupons at the end of the elution experiments.

### IV. Elution Process

Six coated coupons of each drug were used for the elution experiments. The coupons were individually placed, coating side up, in small metal cups to hold the coupon and to allow movement to a new vial at each time point. The coupons were usually placed in a vial containing 10 mL of pH 7.4 phosphate buffered saline. The vials were stored in an orbital shaker, with horizontal shaking of 100 rpm, at 37 °C for at least 30 minutes before insertion of a coupon to allow the solution to equilibrate at the desired temperature. At least nine different time points were observed as shown in Table 10. After the desired time had lapsed, the coupon holder was lifted and allowed to drain. It was then placed into a pre-warmed vial corresponding to the next time point. This procedure continued until the predetermined time had elapsed. At that point, the coupons went through a drug extraction step as outlined earlier. The amount of drug in the elution samples was determined by HPLC.

**Table 10. The 1-Day Elution Study Time and Sample Size Table**

| Sample Number | Elution Time | | Elution Volume |
|---|---|---|---|
| | (Days) | (Hours) | (mL) |
| 1 | 0.003 | 0.08 (5min) | 10 |
| 2 | 0.010 | 0.25 (15 min) | 10 |
| 3 | 0.021 | 0.50 (30 min) | 10 |
| 4 | 0.042 | 1 | 10 |
| 5 | 0.083 | 2 | 10 |
| 6 | 0.125 | 3 | 10 |
| 7 | 0.167 | 4 | 10 |
| 8 | 0.208 | 5 | 10 |
| 9 | 0.250 | 6 | 10 |

To illustrate the effect of a relatively less hydrophilic beneficial agent/hydration inhibitor on a relatively more hydrophilic beneficial agent (i.e., a combination drugs) several different loading procedures were investigated. In particular for ABT-578 and dexamethasone combination the following were investigated.

### Comparative data: Elution profiles

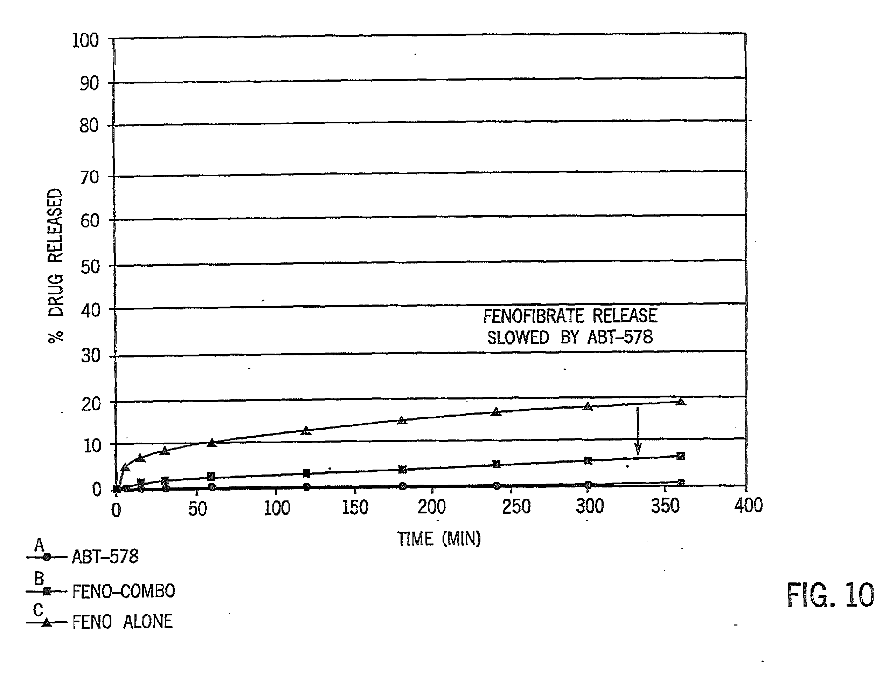

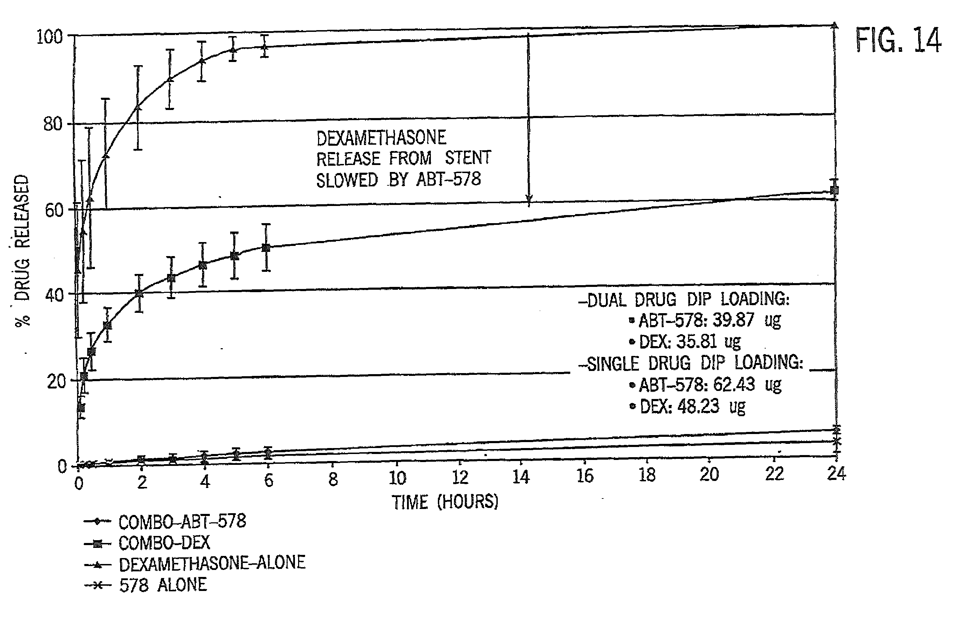

Figures 10, 11, 12, 13 and 14 illustrate the effect of a hydration inhibitor according to the invention on the elution of a relatively more hydrophilic beneficial agent. In Figures 10-13, the drugs were applied to coupons; in Figure 14, stents were coated.

In Figure 10, the six-hour elution profile shown is where the beneficial agent is fenofibrate and the hydration inhibitor is ABT-578. Elution was carried out as described above. Curve A is the elution profile of ABT-578 alone. Curves B and C are the profiles for fenofibrate, in combination with ABT-578 and alone, respectively. Curve B shows that only about 7% of the fenofibrate was released from the coupon after 6 hours. As can be seen by comparing Curves B and C, the release of fenofibrate was significantly reduced by the presence of ABT-578.

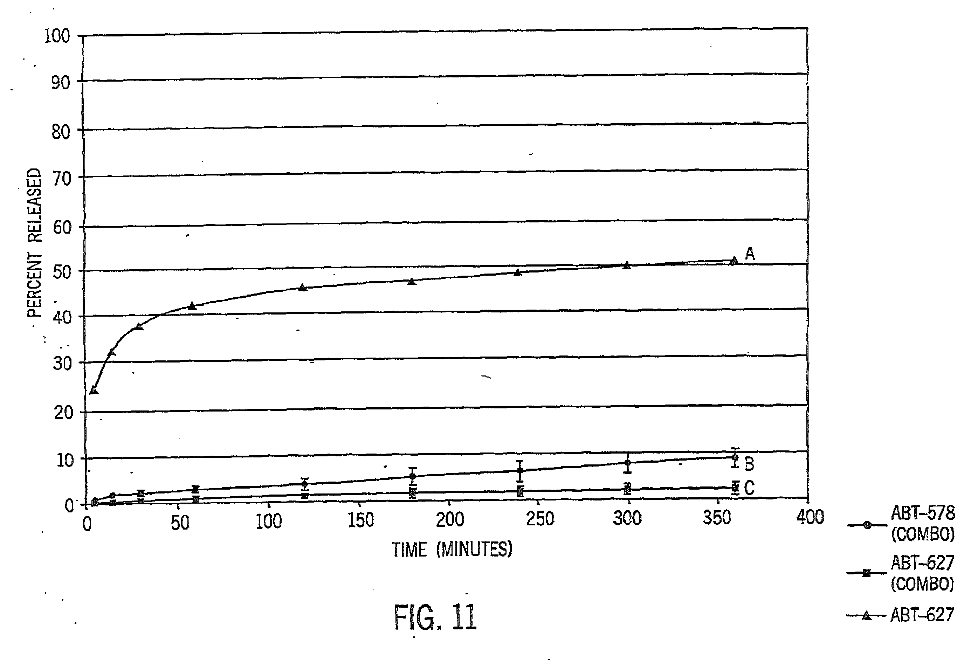

Figure 11 illustrates the six-hour elution profile of beneficial agent ABT-627 (atrasentan) in the presence of hydration inhibitor ABT-578. Curves A and C are the elution profiles of ABT-627, in the presence of ABT-578 and alone, respectively. Curve B shows the elution of ABT-578 under the same conditions. Comparing Curves A and C, it is seen that the elution rate of relatively more hydrophilic ABT-627 is reduced in the presence of relatively less hydrophilic ABT-578. After six hours, much less than 10% of ABT-627 was released in the presence of ABT-578 (Curve C), compared to 50% in the absence of ABT-578 (Curve A).

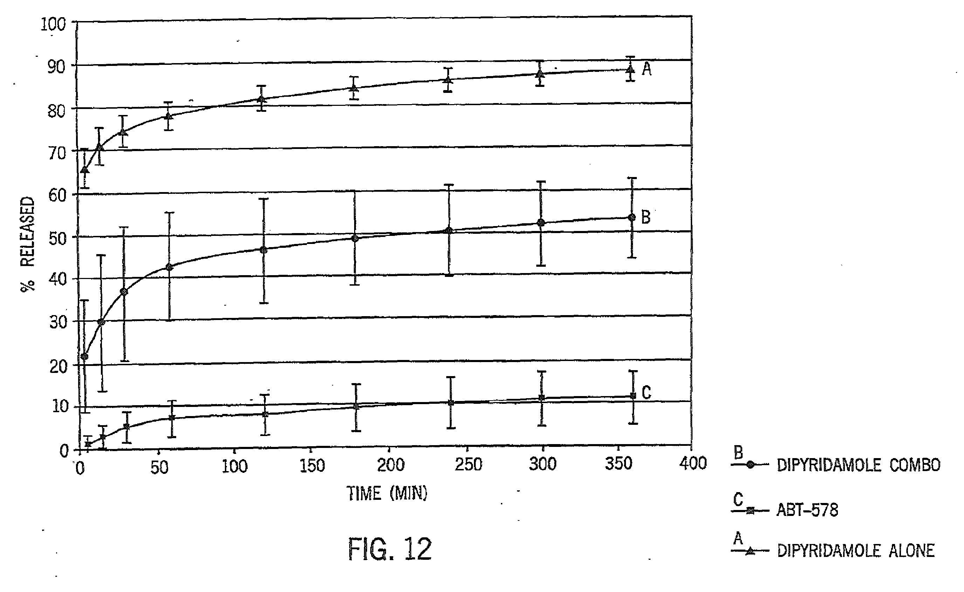

Figure 12 illustrates the six-hour elution profile of beneficial agent dipyridamole in the presence of hydration inhibitor ABT-578. Curves A and B are the elution profiles of dipyridamole, in the presence of ABT-578 and alone, respectively. Curve C shows the elution profile of ABT 578 under the same conditions. As can be seen by comparing Curves A and B, the amount of dipyridamole released from the coupons coated with ABT-578 and dipyridamole is only about 52% after six hours, compared to nearly 90% in the absence of ABT-578.

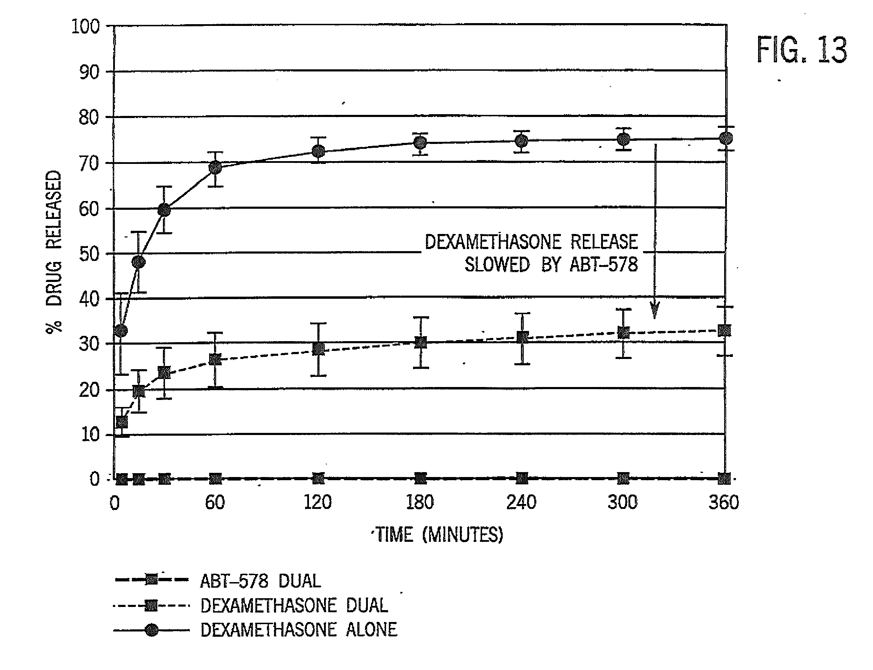

Figure 13 illustrates the six-hour elution profiles of beneficial agent dexamethasone in the presence of hydration inhibitor ABT-578. Curves A and B are the elution profiles of dexamethasone, alone and in the presence of ABT-578, respectively. Curves C and D (superimposed) are the elution profiles for ABT-578, alone and in the presence of dexamethasone, respectively, under the same conditions. As can be seen by comparing Curves A and B, the amount of dexamethasone remaining on the coupon containing dexamethasone and ABT-578 was nearly 70% compared to only 25% on the coupon on which no ABT-578 was present.

Figure 14 illustrates the six-hour elution profile of beneficial agent dexamethasone in the presence of hydration inhibitor ABT-578 on a PC-coated stent. Loading was accomplished by dip loading, that is a stent was dipped into a solution containing either one or both drugs and then permitted to dry. Curves A and B are the elution profiles for dexamethasone in the presence of ABT-578 and alone, respectively. Curves C and D are the elution profiles for ABT-578 in the presence of dexamethasone and alone, respectively. As can be seen by comparing Curves A and B, after 24 hours, almost no dexamethasone was released from the stent containing ABT-578 and dexamethasone, though about 40% of the dexamethasone was released from the stent having no ABT-578 present in the coating.

It is understood that the foregoing detailed description and accompanying examples are merely illustrative and are not to be taken as limitations upon the scope of the invention, which is defined solely by the appended claims and their equivalents. Various changes and modifications to the disclosed embodiments will be apparent to those skilled in the art. Such changes and modifications, including without limitation those relating to the chemical structures, substituents, derivatives, intermediates, syntheses, formulations and/or methods of use of the invention, may be made without departing from the spirit and scope thereof.

## Claims

1. A method of manufacturing a medical device, the method comprising the steps of providing an interventional component to be deployed in a patient; loading a Beneficial agent on the interventional component for delivery therefrom, the beneficial agent having a first LogP value; and
associating an effective amount of a hydration inhibitor with the beneficial agent to control delivery of the beneficial agent front the interventional component, the hydration inhibitor having a second LogP value, the second LogP value being greater than the first LogP value.

2. The method according to claim 1, wherein the Beneficial agent loaded by the loading step is selected from a group consisting of antithrombotics, anticoagulants, antiplatelet agents, anti-lipid agents, thrombolytics, antiproliferatives, anti-inflammatories, agents that inhibit hyperplasia, smooth muscle cell inhibitors, antibiotics, growth factor inhibitors, cell adhesion inhibitors, cell adhesion promoters, antimitotics, antifibrins, antioxidants, antineoplastics, agents that promote endothelial cell recovery, antiallergic substances, viral vectors, nucleic acids, monoclonal antibodies, antisense compounds, oligonucleotides, cell permeation enhancers, and combinations thereof,
particularly a nucleic acid encoding a pharmaceutically useful peptide or an anti-sense oligonucleotide used to control a gene of interest in a cell of the patient, indomethacin, phenyl salicylate, B-estradiol, vinblastine, ABT-627, testosterone, progesterone, paclitaxel, cyclosporin A, vincristine, carvedilol, vindesine, dipyridamole, methotrexatc, folic acid, thrombospondin mimetics, estradiol, dexamethasone, nnetrizamide, iopamidol, iohexol, iopromide, iobitridol, iomeprol, iopentol, ioversol, ioxilan, iodixanol, iotrolan and pro-drugs, analogs, derivatives, or combinations thereof.

3. The method according to claim 2, wherein the hydration inhibitor associated by the associating step is selected from a group consisting of beneficial agents, polymeric materials, markers, additives, and combinations thereof,
particularly a second beneficial agent preferably selected from a group consisting of antithromboties, anticoagulants, antiplatelet agents, anti-lipid agents, thrombolytics, antiprotiferatives, nti-inflammatories, agents that inhibit hyperplasia, smooth muscle cell inhibitors, antibiotics, growth factor inhibitors, cell adhesion inhibitors, cell adhesion promoters, antimitotics, antifibrins, antioxidants, antincoplastics, agents that promote endothelial cell recovery, antiallergic substances, viral vectors, nucleic acids, monoclonal antibodies, antisense compounds, oligonucleolides, cell permeation enhancers, and combinations thereof,
particularly paclitaxel, rapamycin, rapamycin derivatives, pimecrolimus, everolimus, fenofibratc, carvedilol, taxcoteres, tacrolimus, butylated hydroxytoluene, butylated hydroxyanisole,
vitamin E, danazol, probucol, tocopherols, tocotriertols, ABT-578, ABT-627 and analogs, derivatives, or combinations thereof.

4. The method according to claim 1, wherein the associating step includes applying the second beneficial agent as a layer to at least partially cover the first beneficial agent.

5. The method according to claim 4, further comprising the step of applying a third layer of a third beneficial agent on at least a portion of the interventional component, the third beneficial agent having a third LogP value.

6. The method according to claim 5, wherein the third LogP value is greater than the second LogP value, or wherein the third LogP value is the same as the first Log P value.

7. The method according to claim 4, wherein the associating step includes forming a mixture of the second beneficial agent with the first beneficial agent, or
wherein the associating step includes forming a mixture of the hydration inhibitor and the beneficial agent,
particularly the hydration inhibitor associated by the associating stop is an additive, wherein the additive is preferably selected from a group consisting of nitrophenyl octyl ether, bisethylhexyl sebacate, diisododecylphthalate, N-methylpyrrolidone, linolenic acid, linoleic acid stearic acid, oleic acid, and combinations thereof, and/or
the hydration inhibitor associated by the association step is a polymeric material, wherein the polymeric material is preferable selected from a group consisting of phosphorylcholine, polycaprolactone, poly-D,L-lactic acid, poly-L-lactic acid, poly(lactide-co-glycolide), poly(hydroxybutyrate), poly(hydroxybutyrate-co-valerate), polydioxanone, polyorthoester, polyanhydridc, poly(glycolic acid), poly(glycolic aoid-co-trimethylene carbonate), polyphosphoester, polyphosphoester urethane, poly(amino acids), cyanoacrylates, poly(trimethylene carbonate), poly(iminocarbonate), polyalkylene oxalates, polyphosphazenes, polyiminocarbonates, and aliphatic polycarbonates, fibrin, fibrinogen, cellulose, starch, collagen, Parylene®, Parylast®, polyurethane, polycarbonate urethanes, polyethylene, polyethylene terephthalate, ethylene vinyl acetate. ethylene vinyl alcohol., silicone polysiloxanes, substituted polysiloxanes, polyethylene oxide, polybutylene terephthalate-co-PEG, PCL-co-PEG, PLA-co-PL-G, polyacrylates, polyvinyl pyrrolidone, polyacrylamide, thermoplastic elastomers, polyolefin elastomers, EPDM rubbers, polyamide elastomers, biostable plastic, acrylic polymers, nylon, polyesters, epoxies, and derivatives or combination thereof, or
wherein the polymeric material has a zwitterionic pendant group.

8. The method according to claim 1, further comprising the step of applying a layer of polymeric material on at least a portion of a surface of the interventional component, and further wherein the loading step includes loading the beneficial agent at least partially onto the layer of polymeric material particularly
wherein the layer of polymeric material applied by the applying step has a zwitterionic pendant group, preferably a phosphoryl choline pendant group, and/or
wherein the hydration inhibitor associated by the associating step controls a delivery of the beneficial agent from the layer of polymeric material.

9. The method according to claim 1, wherein the interventional component is selected from the group consisting of a stent, graft, stent-graft, valve, filter, coil, staple, suture, guidewire, and catheter.

10. The method according to claim 1, wherein the first LogP value of the beneficial agent loaded by the loading stop is at least about 0.5 units less than the second LogP value of the hydration inhibitor associated by the associating step.

11. The method according to claim 1, wherein the effective amount of hydration inhibitor is an amount sufficient to shift the liquid-solid contact angle of the beneficial agent in association with the hydration inhibitor to at least 50°, wherein the liquid-solid contact angle of the beneficial agent in association with the hydration inhibitor is preferably at least about 70°.
